# NEW EUROPEAN PATENT SPECIFICATION

(11) **EP 1 913 157 B2**
(45) Date of publication and mention of the opposition decision: **26.10.2016**
(45) Mention of the grant of the patent: 28.11.2012
(21) Application number: 06773201.6
(22) Date of filing: 13.06.2006
(51) Int. Cl.: C12Q 1/68

(54) **EGFR AND KRAS MUTATIONS FOR PREDICTION OF PATIENT RESPONSE TO EGFR INHIBITOR TREATMENT**
EGFR- UND KRAS-MUTATIONEN ZUR VORHERSAGE DER REAKTION EINES PATIENTEN AUF EGFR-HEMMER-BEHANDLUNG
MUTATIONS CHEZ EGFR ET KRAS POUR PREDIRE LA REPONSE D'UN PATIENT AU TRAITEMENT AVEC UN INHIBITEUR D'EGFR

(30) Priority: 28.06.2005 US 695174 P
(43) Date of publication of application: 23.04.2008
(73) Proprietor: Genentech, Inc., South San Francisco, CA 94080-4990 (US)
(72) Inventor: HILLAN, Kenneth, J., San Francisco, CA 94114 (US)
(74) Representative: Denison, Christopher Marcus
(86) International application number: PCT/US2006/023230
(87) International publication number: WO 2007/001868

(56) References cited:
- WO-A-2004/111273
- WO-A-2005/118876
- EBERHARD ET AL: "Correlation of mutations in EGFR with clinical outcomes in NSCLC patients treated with erlotinib" EUROPEAN JOURNAL OF CANCER SUPPLEMENTS, PROCEEDINGS OF THE 16TH EORTC-NCI-AACR SYMPOSIUM ON MOLECULAR TARGETS AND CANCER THERAPEUTICS, vol. 2, no. 8, September 2004 (2004-09), page 124, XP002400934
- PAO WILLIAM ET AL: "KRAS mutations and primary resistance of lung adenocarcinomas to gefitinib or erlotinib." PLOS MEDICINE. JAN 2005, vol. 2, no. 1, January 2005 (2005-01), page e17, XP002400935 ISSN: 1549-1676
- MORONI M ET AL: "Gene copy number for epidermal growth factor receptor (EGFR) and clinical response to antiEGFR treatment in colorectal cancer: a cohort study" LANCET ONCOLOGY, LANCET PUBLISHING GROUP, LONDON, GB, vol. 6, no. 5, May 2005 (2005-05), pages 279-286, XP004870903 ISSN: 1470-2045
- AMLER L C ET AL: "Predicting clinical benefit in non-small-cell lung cancer patients treated with epidermal growth factor tyrosine kinase inhibitors." COLD SPRING HARBOR SYMPOSIA ON QUANTITATIVE BIOLOGY. 01-06 JUNE 2005, vol. 70, 2005, pages 483-488, XP008069387 ISSN: 0091-7451
- EBERHARD DAVID A ET AL: "Mutations in the epidermal growth factor receptor and in KRAS are predictive and prognostic indicators in patients with non-small-cell lung cancer treated with chemotherapy alone and in combination with erlotinib." JOURNAL OF CLINICAL ONCOLOGY : OFFICIAL JOURNAL OF THE AMERICAN SOCIETY OF CLINICAL ONCOLOGY. 1 SEP 2005, vol. 23, no. 25, 1 September 2005 (2005-09-01), pages 5900-5909, XP002400936 ISSN: 0732-183X
- EBERHARD ET AL: "O-186 Mutations in EGFR, HER2, KRAS and BRAF in NSCLC: Prevalences and correlations with clinical outcomes in patients treated with carboplatin and paclitaxel with or without erlotinib" LUNG CANCER, ELSEVIER, AMSTERDAM, NL, vol. 49, July 2005 (2005-07), page S62, XP005048768 ISSN: 0169-5002
- RECK ET AL: "PD-155 Molecular markers such as EGFR and kRAS mutations aspredictors of sensitivity to erlotinib in patients (pts) with NSCLC: Exploratory subanalyses of TALENT, a phase III trial" LUNG CANCER, ELSEVIER, AMSTERDAM, NL, vol. 49, July 2005 (2005-07), page S112, XP005048936 ISSN: 0169-5002
- LIÈVRE ASTRID ET AL: "KRAS mutation status is predictive of response to cetuximab therapy in colorectal cancer." CANCER RESEARCH. 15 APR 2006, vol. 66, no. 8, 15 April 2006 (2006-04-15), pages 3992-3995, XP002400937 ISSN: 0008-5472
- OGINO SHUJI ET AL: "Molecular alterations in tumors and response to combination chemotherapy with gefitinib for advanced colorectal cancer." CLINICAL CANCER RESEARCH : AN OFFICIAL JOURNAL OF THE AMERICAN ASSOCIATION FOR CANCER RESEARCH. 15 SEP 2005, vol. 11, no. 18, 15 September 2005 (2005-09-15), pages 6650-6656, XP002400938 ISSN: 1078-0432
- SUZUKI TAKESHI ET AL: "The sensitivity of lung cancer cell lines to the EGFR-selective tyrosine kinase inhibitor ZD1839 ('Iressa') is not related to the expression of EGFR or HER-2 or to K-ras gene status", LUNG CANCER, ELSEVIER, AMSTERDAM, NL LNKD- DOI:10.1016/S0169-5002(03)00278-2, vol. 42, no. 1, 1 October 2003 (2003-10-01), pages 35-41, XP002564069, ISSN: 0169-5002
- SHIGEMATSU HISAYUKI ET AL: "Somatic mutations of the HER2 kinase domain in lung adenocarcinomas", CANCER RESEARCH, AMERICAN ASSOCIATION FOR CANCER REREARCH, US LNKD- DOI:10.1158/0008-5472.CAN-04-4235, vol. 65, no. 5, 1 March 2005 (2005-03-01), pages 1642-1646, XP002350900, ISSN: 0008-5472
- D. STEWART ET AL.: 'Molecular approaches to controlling cancer', June 2005, COLD SPRING HARBOR, NEW YORK pages V - VI - 303-304

## Description

### FIELD OF THE INVENTION

The present invention relates to cancer diagnostics and therapies and in particular to the detection of mutations that are diagnostic and/or prognostic.

### BACKGROUND OF THE INVENTION

Epidermal Growth Factor Receptor (EGFR) is a member of the type 1 tyrosine kinase family of growth factor receptors, which play critical roles in cellular growth, differentiation, and survival. Activation of these receptors typically occurs via specific ligand binding, resulting in hetero- or homodimerization between receptor family members, with subsequent autophosphorylation of the tyrosine kinase domain. This activation triggers a cascade of intracellular signaling pathways involved in both cellular proliferation (the ras/raf/MAP kinase pathway) and survival (the PI3 kinase/Akt pathway). Members of this family, including EGFR and HER2, have been directly implicated in cellular transformation.

A number of human malignancies are associated with aberrant or overexpression of EGFR and/or overexpression of its specific ligands e.g. transforming growth factor α (Gullick, Br Med Bull 1991, 47:87-98; Modijtahedi and Dean, Int J Oncol 1994, 4:277-96; Salomon et al., Crit Rev Oncol Hematol 1995;19:183-232). EGFR overexpression has been associated with an adverse prognosis in a number of human cancers, including NSCLC. In some instances, overexpression of tumor EGFR has been correlated with both chemoresistance and a poor prognosis (Lei et al., Anticancer Res 1999;19:221-8; Veale et al., Br J Cancer 1993;68:162-5). These observations suggest that agents that effectively inhibit EGFR receptor activation and subsequent downstream signaling may have clinical activity in a variety of human cancers, including NSCLC.

Tarceva™ (also known as erlotinib; OSI-774), a quinazoline, is an orally active, potent, selective inhibitor of EGFR tyrosine kinase. Erlotinib inhibits human EGFR tyrosine kinase with an IC₅₀ of 2 nM (0.786 mg/mL) in an in vitro enzyme assay. This inhibition is selective for EGFR tyrosine kinase, results in cell cycle arrest at G₁, and is reversible. Oral administration of erlotinib in mice has demonstrated a >70% reduction in EGFR autophosphorylation in human xenografts and marked growth inhibition of HN5 and A431 xenografts in nude mice has been demonstrated. In addition to single-agent activity in in vivo assay systems, erlotinib has been evaluated in combination with a number of chemotherapy agents to determine possible interactions. There was an additive interaction between erlotinib and paclitaxel, cisplatin, gemcitabine, and doxorubicin.

Lung cancer represents the leading cause of cancer-related mortality for both men and women in the United States. In 2000, it was estimated that 164,000 new cases would be diagnosed and 157,000 patients would die from this disease (Greenlee et al., CA Cancer J Clin 2001, 51:15-36). Approximately 75% of these patients would have had non-small cell histologies, with the majority presenting with inoperable Stage IIIB or Stage IV disease. For those patients with more limited disease at presentation (Stages I-IIIA), relapse following standard surgical therapy, with or without adjuvant or neoadjuvant chemo- and/or radiotherapy, is common. These findings result in an overall 5-year survival in non-small cell lung cancer (NSCLC) of ∼12% and serve to emphasize the unmet medical need in this disease.

The platinum compound cisplatin was the first chemotherapy agent to show clinical benefit in the management of locally advanced or metastatic NSCLC. Randomized clinical trials demonstrated improved response rates, quality of life, and survival compared with the best supportive care (Rapp et al. 1988). However, the magnitude of this improvement was modest-measured in weeks. Subsequently, a number of newer chemotherapy agents have been evaluated as single agents and in combination with the platinum salts in the first-line setting. The conclusion from these studies is that modem "doublet" chemotherapy appears to achieve response rates of 15%-20%, median time to disease progression of 3-4 months, and median survival of 7-8 months. The modest improvements in efficacy with combination therapies over the results obtained with cisplatin have established these therapies as a standard of care for patients with advanced NSCLC and an acceptable performance status (Non-Small Cell Lung Cancer Cooperative Group, Br Med J 1995, 311:899-909; American Society of Clinical Oncology, J Clin Oncol 1997, 15:2996-3018; Breathnach et al., J Clin Oncol 2001;19: 1734-42).

PAO W. et al. (KRAS mutations and primary resistance of lung adenocarcinomas to gefitinib or erlotinib. PLOS MEDICINE. vol. 2, no. 1, January 2005 (2005-01), page e17) discloses that mutations in Kras are associated with a lack of sensitivity to gefitinib or erlotinib, while mutations in EGFR are associated with sensitivity to said molecules, and proposes to improve treatment decisions by determining the mutational status of both EGFR and Kras.

### SUMMARY OF THE INVENTION

The invention provides a method of identifying a patient nonresponsive to treatment with an EGFR inhibitor in combination with a chemotherapeutic agent, as defined in the claims.

Disclosed herein is a method for identifying a tumor in a human subject that is susceptible to treatment comprising determining the presence of a mutated EGFR gene or mutated EGFR protein in a sample of said tumor wherein said mutation is located in exons 18-21 of EGFR whereby the presence of a mutated EGFR gene or mutated EGFR protein indicates the tumor is susceptible to treatment

Disclosed herein is a method of treating a tumor in a mammal comprising identifying the presence of an EGFR mutation in said tumor and treating said mammal with an anticancer agent.

Disclosed herein is a method of identifying an EGFR mutation in a sample comprising contacting nucleic acid from said sample with a probe that is capable of specifically hybridizing to nucleic acid encoding a mutated EGFR protein, or fragment thereof incorporating a mutation, and detecting the hybridization.

Disclosed herein are nucleic acid probes capable of specifically hybridizing to nucleic acid encoding a mutated EGFR protein or fragment thereof incorporating a mutation.

Disclosed herein is a a method of detecting a mutated EGFR gene in a sample comprising amplifying from said sample nucleic acid corresponding to the kinase domain of said EGFR gene, or a fragment thereof suspected of containing a mutation, and comparing the electrophoretic mobility of the amplified nucleic acid to the electrophoretic mobility of corresponding wild-type EGFR gene or fragment thereof.

Disclosed herein is a a method for identifying a tumor in a human subject that is susceptible to treatment with an EGFR inhibitor comprising (i) determining the presence of a wild-type KRAS protein or gene in a sample of said tumor whereby the presence of a wild-type KRAS protein or gene indicates that the tumor is susceptible to treatment with an EGFR inhibitor or (ii) determining the presence of a mutated KRAS protein or gene in a sample of said tumor whereby the absence of a mutated KRAS protein or gene indicates that the tumor is susceptible to treatment with an EGFR inhibitor.

### BRIEF DESCRIPTION OF THE DRAWINGS

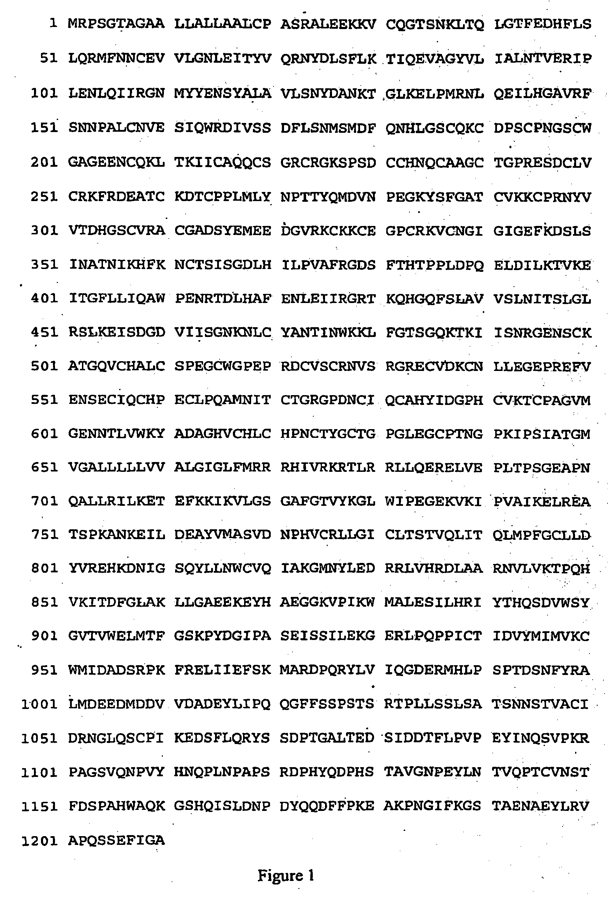

Figure 1 illustrates the amino acid sequence of wild-type EGFR1 (SEQ ID NO: 1) in which the signal sequence is residues 1-24, the extracellular domain includes residues 24-645, the transmembrane domain includes residues 646-668, and the cytoplasmic domain includes residues 669-1210. The tyrosine kinase domain region is residues 718-964, and the threonine phosphorylation site is residue 678.
Figure 2a through 2d is the cDNA sequence (SEQ ID NO: 2) of wild-type EGFR in which exon 18 corresponds to nucleotides 2308-2430; exon 19 corresponds to nucleotides 2431-2529; exon 20 corresponds to nucleotides 2530-2715 and exon 21 corresponds to 2716-2871.

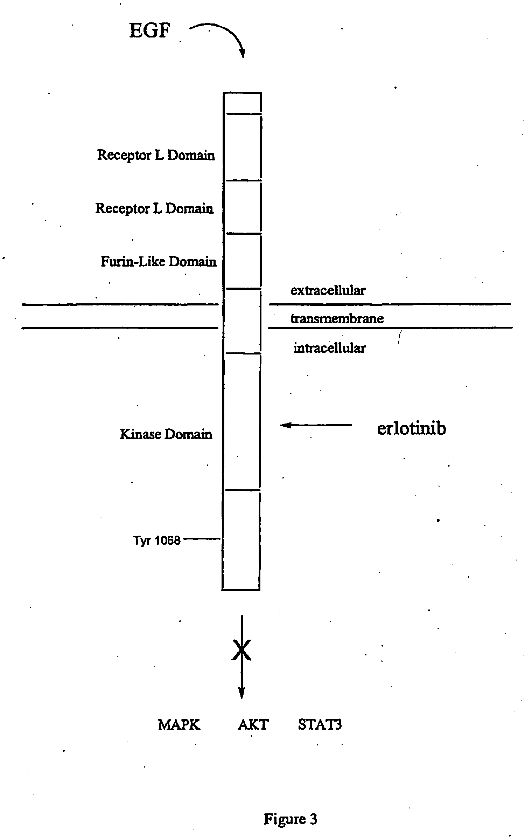

Figure 3 is a graphical representation of extracellular (top) and intracellular (bottom) regions of EGFR.

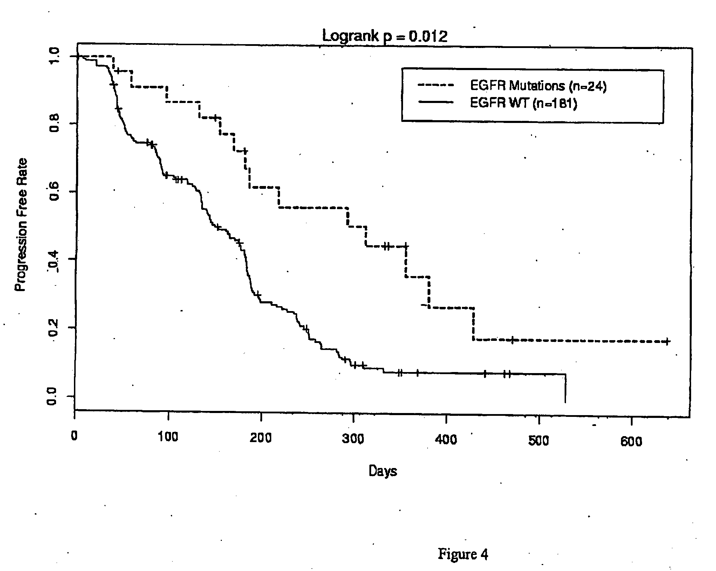

Figure 4 is a Kaplan-Meier curve showing time to progression of patients having NSCLC tumors expressing wild-type EGFR (solid line) and mutant EGFR (dashed line).

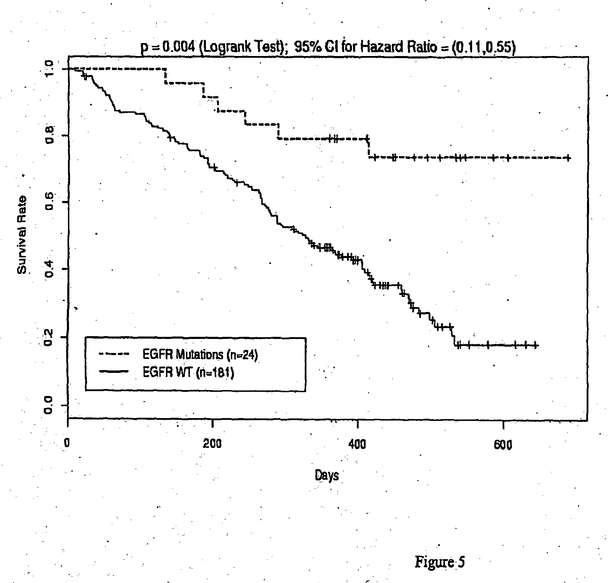

Figure 5 is a Kaplan-Meier curve showing survival of patients having NSCLC tumors expressing wild-type EGFR (solid line) and mutant EGFR (dashed line).

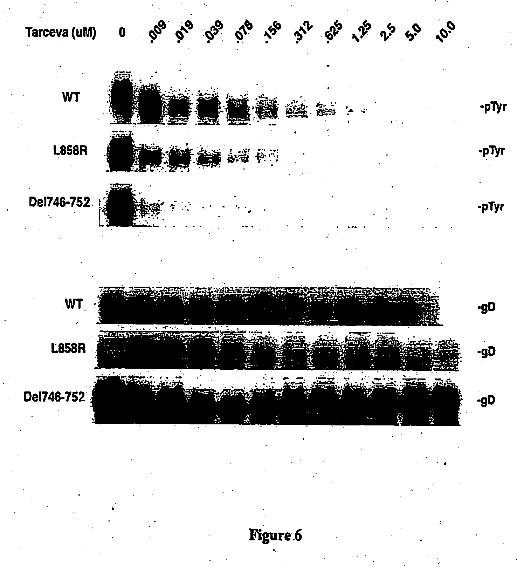

Figure 6 is an autoradiograph illustrating inhibition of autophosphorylation of wild-type EGFR, and mutant EGFR (L858R and de1746-752) with varying concentrations of erlotinib in transiently transfected COS7 cells.

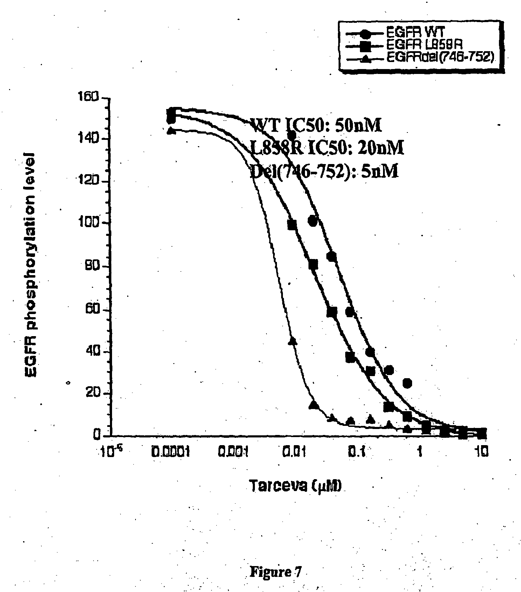

Figure 7 is a graph showing inhibition of autophosphorylation of wild-type EGFR and mutant EGFR (L858R and de1746-752) with varying concentrations of erlotinib in transiently transfected COS7 cells.

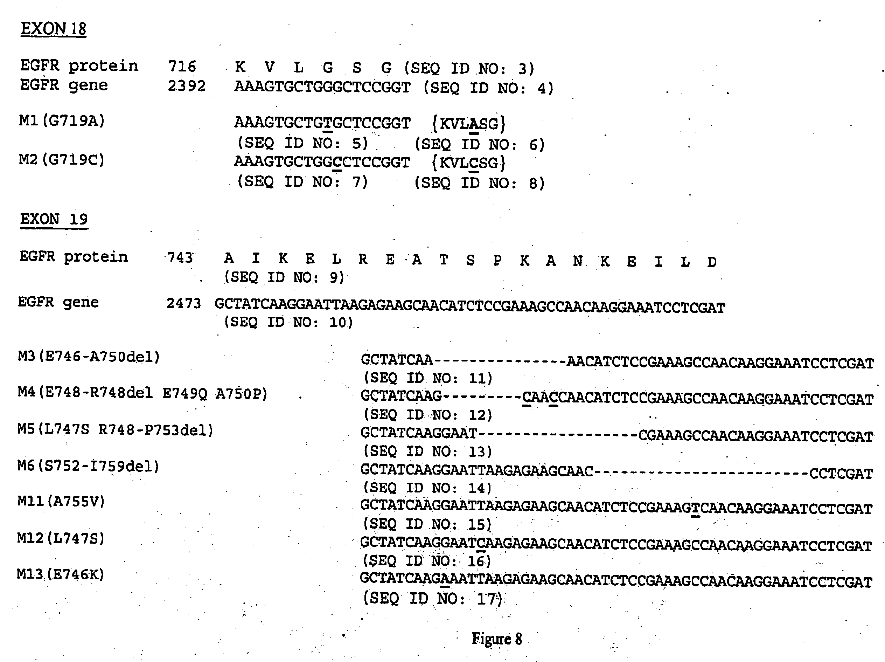

Figure 8 illustrates mutations in exons 18 and 19 of EGFR gene and protein sequences. Amino acid and nucleotide changes, and insertions are in bold, underlined font while deletions are shown as dashes (-).

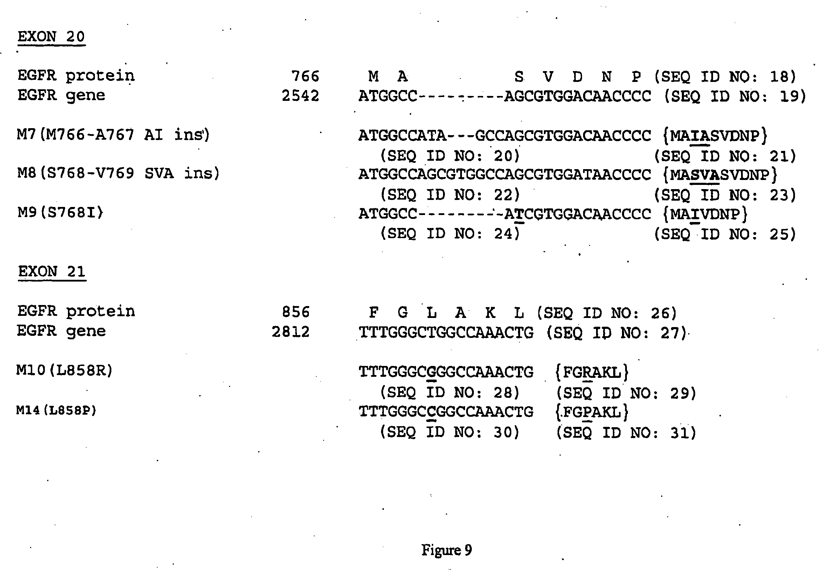

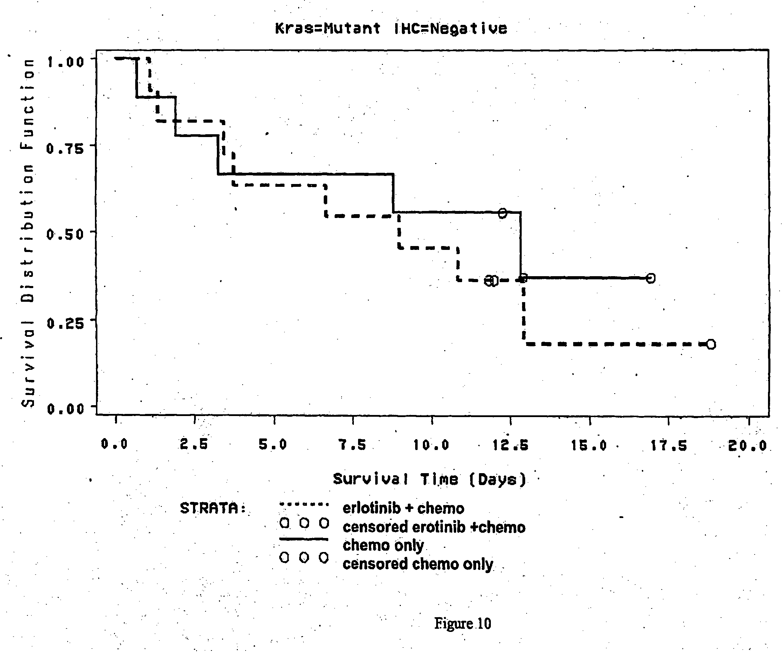

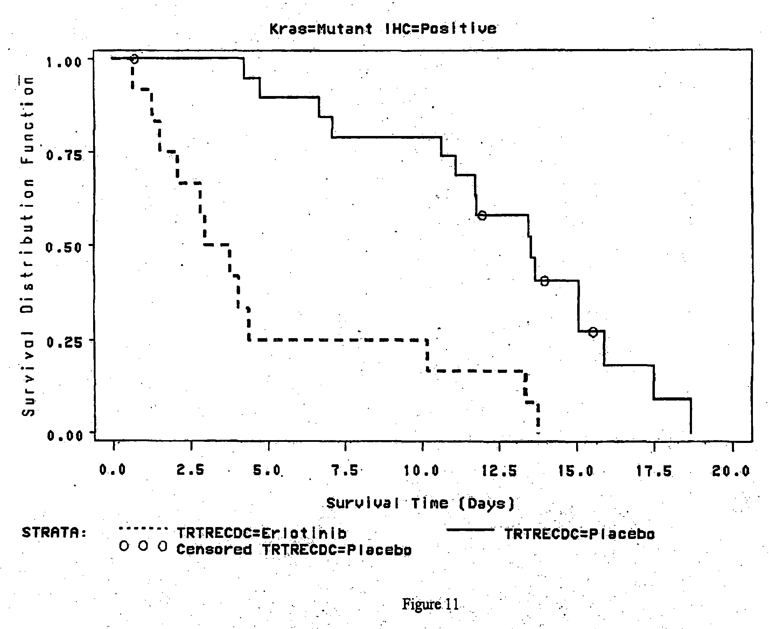

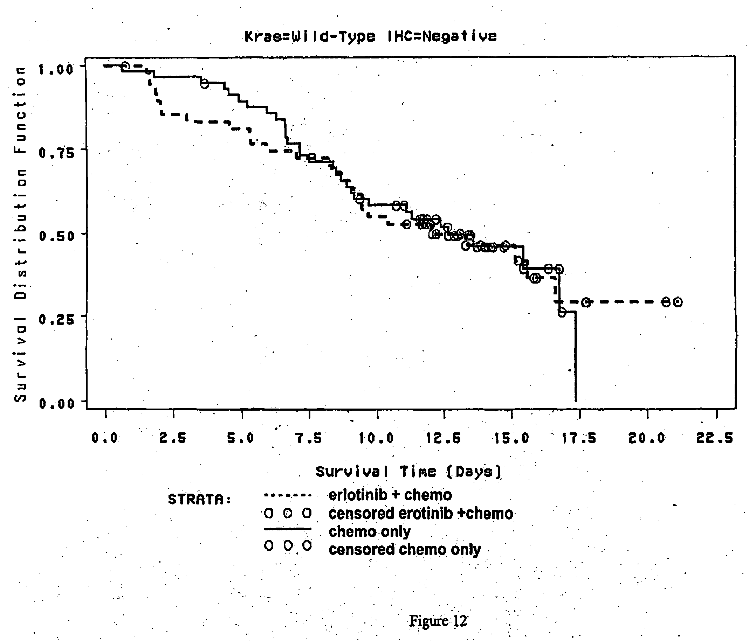

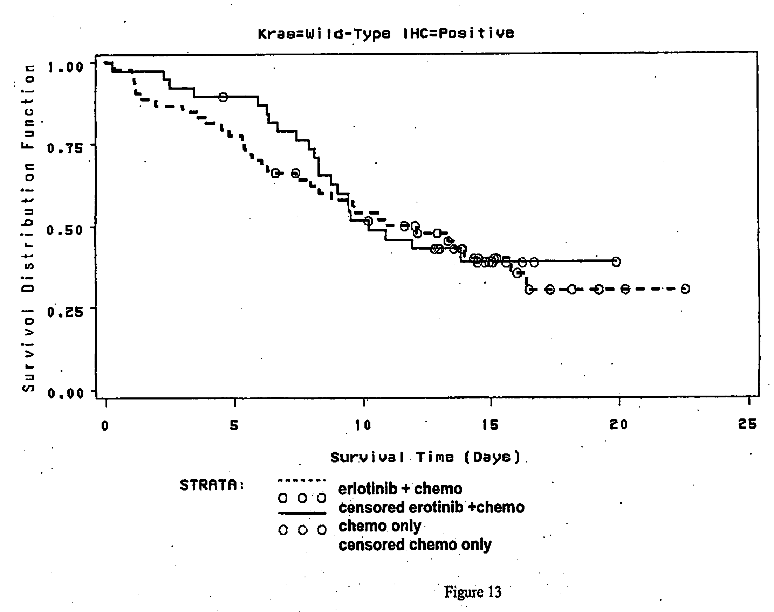

Figure 9 illustrates mutations in exons 20 and 21 of EGFR gene and protein sequences. Amino acid and nucleotide changes, and insertions are in bold, underlined font while deletions are shown as dashes (-).

### DETAILED DESCRIPTION OF THE PREFERRED EMBODIMENTS

It is a discovery of the present disclosure that mutational events associated with tumorigenesis occur in Epidermal Growth Factor Receptor (EGFR). Although it was previously known that aberrant EGFR activity was associated with various cancers, it was unknown that mutations in the EGFR kinase domain region (KDR) existed that caused aberrant signaling activity associated with some cancers. Surprisingly patients suffering from tumors having EGFR KDR mutations have a better prognosis than those with wild-type EGFR. The KDR mutations of the EGFR gene can involve rearrangements such as insertions and deletions as well as point mutations.

Samples from approximately 250 patients who participated a randomized, double-blinded phase III clinical trial referred to as Tribute were sequenced for mutations occurring in exons 18-21 of EGFR. Tribute studied 1,079 patients at approximately 150 centers in the United States having histological confirmed NSCLC who had not received prior chemotherapy comparing erlotinib + chemotherapy (carboplatin/paclitaxel) with chemotherapy alone. Patients received paclitaxel (200 mg/m² 3 hour i.v. infusion) followed by carboplatin (AUC = 6 mg/ml x minute infused over 15-30 minutes using Calvert formula) with or without erlotinib (100 mg/day p.o. escalated to 150mg/day for tolerant patients). Tumor samples, formalin-fixed paraffin-embedded blocks or unstained slides, from approximately 250 patients collected from the Tribute trial were enriched for tumor cells by laser capture mircrodissection followed by DNA extraction. Exons 18-21 were amplified by nested PCR and bi-directional sequences were obtained from each PCR product using fluorescent dye-terminator chemistry. Mutations discovered from the sequencing are shown in table 1:

**Table 1**

| protein mutation | nucleic acid mutation | exon |
|---|---|---|
| G719A | 2402G>C | 18 |
| G719C | 2401G>T | 18 |
| G719S | 2401G>A | 18 |
| E746-R748 del | 2482-2490 del GGAATTAAGA (SEQ ID NO: 32) | 19 |
| E746-A750 del | 2481-2495 del GGAATTAAGAGAAGC (SEQ ID NO: 33) | 19 |
| E746-R748 del | 2482-2490 del GAATTAAGA | |
| E749Q | 2491G>C | |
| A750P | 2494G>C | 19 |
| L747-E749 del | 2485-2493 del TTAAGAGAA | |
| A750P | 494G>C | 19 |
| L747S | | |
| R748-P753 del | 2486-2503 del TAAGAGAAGCAACATCTC (SEQ ID NO: 34) | 19 |
| L747-S752 del E746V | 2485-2502 del TTAAGAGAAGCAACATCT 2483A>T (SEQ ID NO: 35) | 19 |
| L747-T751 del ins S | 2486-2494del TAAGAGAAGCAA (SEQ ID NO: 36) | 19 |
| S752-I759 del | 2499-2522 del ATCTCCGAAAGCCAACAAGGAAAT (SEQ ID NO: 37) | 19 |
| M766-A767 AI ins | 2544-2545 ins GCCATA | 20 |
| S768-V769 SVA ins | 2554-2555 ins CCAGCGTGG (2556C>T silent) | 20 |
| L858R | 2819T>G | 21 |
| G719C | 2401G>T | 18 |
| S7681 | 2549G>T {2607G>A SNP silent} | 20 |
| G719C | 2401G>T | 18 |
| V765M | 2539G>A | 20 |
| S7681 | 2549G>T | 20 |
| A755V | 2510C>T | 19 |
| L747S | 2486T>C | 19 |
| E746K | 2482G>A | 19 |
| P772-H773 V ins | 2561-2562 ins GGT | 20 |
| L858P | 2819T>C | 21 |
| L861Q | 2576T>A | 21 |
| P772-H773 NS ins | 2562-2563 ins AACTCC | |
| H773Y | 2563C>T | 20 |
| T790M | 2615C>T | 20 |
| L858R | 2819T>G | 21 |
| S784F | | 21 |
| L858R | | 21 |
| ins = insertion del = deletion | | |

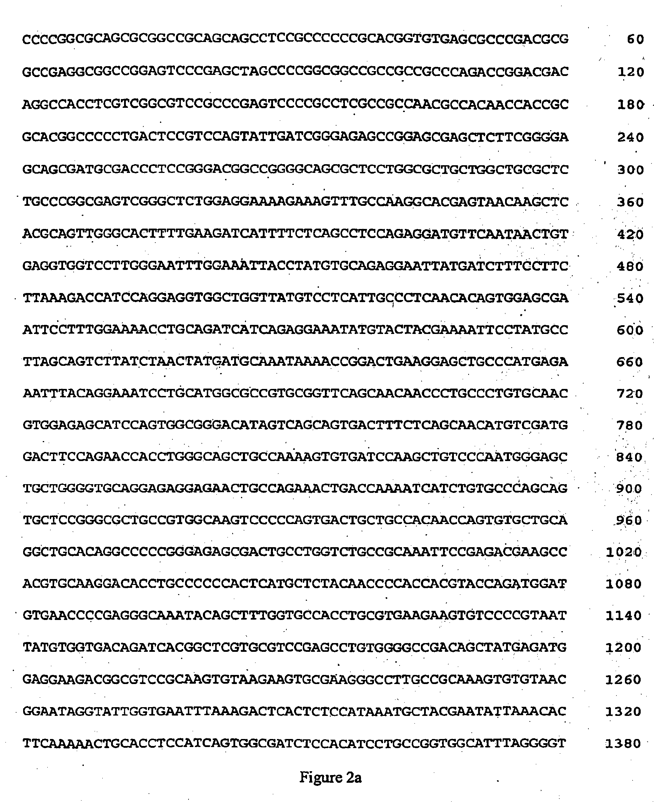

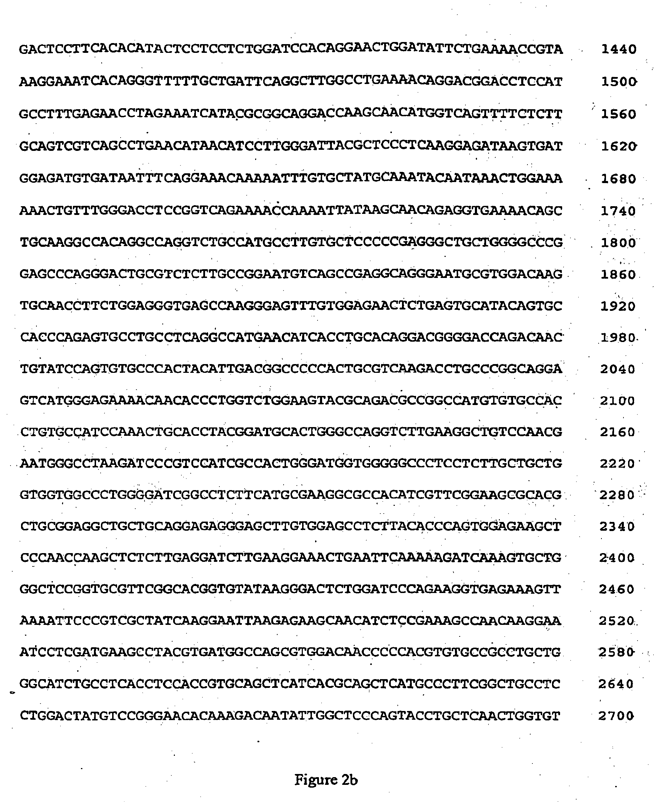

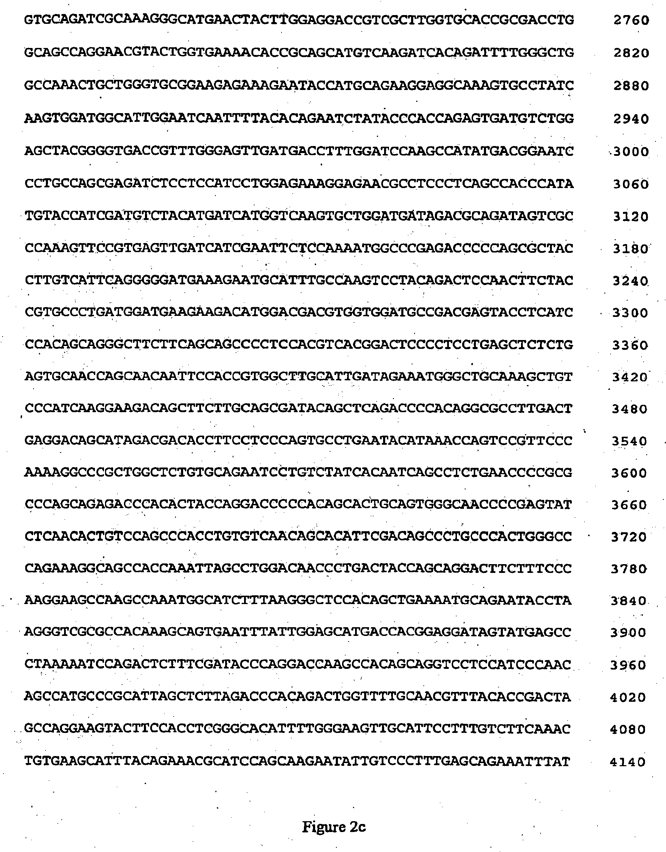

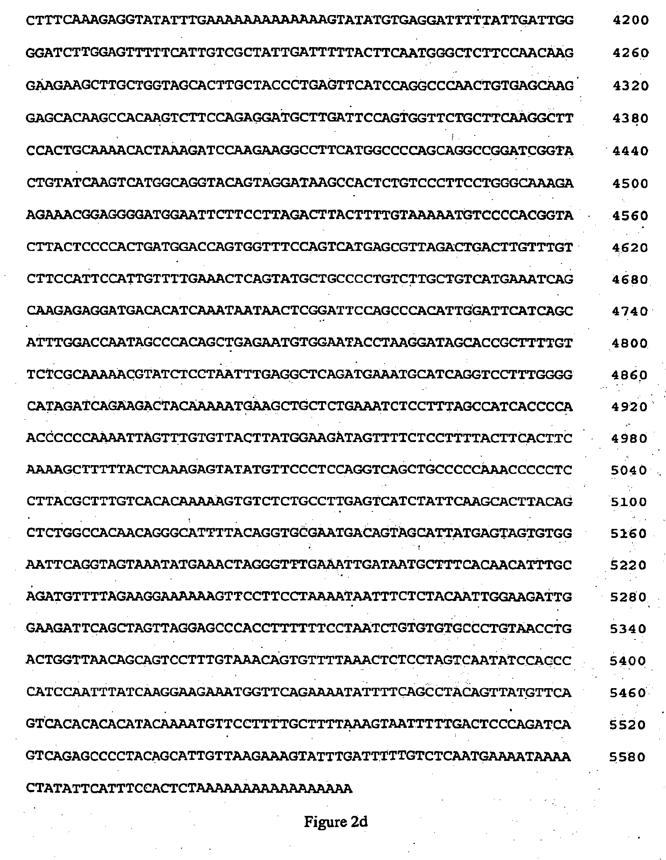

Nucleotide numbering for mutations is based on reference sequence shown in figures 2a-2d.

Clinical outcome of patients having tumors with EGFR mutations and wild-types EGFR were analyzed according to response (complete + partial) benefit (response + stable disease) and progressive disease. Lesions were evaluated using Response Evaluation Criteria in Solid Tumors (RECIST) criteria whereby "complete response" (CR) is defined as the disappearance of all target lesions; "partial response" (PR) is defined as at least a 30% decrease in the sum of the longest diameter of target lesions, taking as reference the baseline sum longest diameter; "progressive disease" (PD) is defined as at least a 20% increase in the sum of the longest diameter of target lesions, taking as reference the smallest sum longest diameter recorded since the treatment started or the appearance of one or more new lesions; and "stable disease" (SD) is defined as neither sufficient shrinkage to qualify for partial response nor sufficient increase to qualify for progressive disease, taking as reference the smallest sum longest diameter since the treatment started.

Results of the analysis are summarized in table 2.

**Table 2**

| | Mutant EGFR n=24 | | Wild-Type EGFR n=181 | |
|---|---|---|---|---|
| Response / Benefit Rate | | | | |
| response (CR + PR) | 11 | 46% | 46 | 25% |
| benefit (CR + PR + SD) | 18 | 75% | 105 | 58% |
| SD | 7 | 29% | 59 | 33% |
| PD | 6 | 25% | 76 | 42% |
| Survival (days) | | | | |
| median | 435 | | 309 | |
| range | 133-687 | | 9-643 | |
| CR=complete response; PR=partial response; SD=stable disease; PD=progressing disease | | | | |

Analysis of clinical outcome revealed that patients with tumors expressing a mutation in exons 18-21 of EGFR have better prognosis than those with tumors expressing wild-type EGFR. Mutant EGFR patients exhibited greater response rate, benefit rate and survival when treated with chemotherapy or chemotherapy plus erlotinib. These results are useful for predicting outcome such that patients whose tumors have EGFR mutations in any or all of exons 18 through 21 have more favorable prognosis than patients whose tumors do not have such mutations.

Accordingly, the present disclosure provides a method for determining the prognosis of a patient having a tumor comprising determining in a sample of said tumor the presence or absence of one or more EGFR mutations in exons 18-21 (or the amino acid sequence corresponding to exons 18-21) whereby the presence of said one or more EGFR mutation indicates better prognosis compared to the absence of said one or more EGFR mutation. By "prognosis" is meant response and/or benefit and/or survival. By "EGFR mutations" means an amino acid or nucleic acid sequence that differs from wild-type EGFR protein or nucleic acid respectively found on one allele (heterozygous) or both alleles (homozygous) and may be somatic or germ line. In a particular embodiment said mutation is found in the kinase domain region (KDR) of EGFR. In another particular embodiment the mutation is an amino acid substitution, deletion or insertion as shown in table 1. In an embodiment the amino acid mutation is one or more of the following: G719A, E746K, L747S, E749Q, A750P, A755V, S768I, L858P, E746-R748 del, R748-P753 del, M766-A767 AI ins, and S768-V769 SVA ins. In another particular embodiment, the mutation is a nucleic acid point mutation, deletion or insertion as shown in table 1. In an embodiment, the nucleic acid mutation is one or more the following: 2402G>C; 2482G>A; 2486T>C; 2491G>C; 2494G>C; 2510C>T; 2549G>T; 2819T>C; 2482-2490 del; 2486-2503 del; 2544-2545 ins GCCATA; and 2554-2555 ins CCAGCGTGG.

EGFR exons 18-21 from an H1975 tumor cell line that exhibited resistance to treatment with erlotinib was sequenced and found to incorporate a mutation T790M in combination with an L858R mutation. Accordingly the present disclosure further provides a method for determining the prognosis of a patient having a tumor comprising determining in a sample of said tumor the presence or absence of the T790M EGFR mutation whereby the presence of said T790M EGFR mutation indicates poorer prognosis compared to the absence of said T790M EGFR mutation. Further, there is provided a method of identifying patients having a tumor that is less responsive to therapy of an EGFR inhibitor such as erlotinib or gefitinib, whether in combination with chemotherapy or not, comprising determining the presence or absence of a T790M EGFR mutation in the patient's tumor whereby the presence of said mutation indicates the patient will respond less to said therapy compared to a patient having a tumor that does not have said T790M EGFR mutation. Further, there is provided a method of identifying a tumor that is resistant to treatment with an EGFR inhibitor, such as a kinase domain binding inhibitor (for example erlotinib or gefitinib), whether in combination with chemotherapy or not, comprising determining the presence or absence of a T790M EGFR mutation in a sample of the tumor whereby the presence of said mutation indicates the tumor is resistant to said treatment. It is understood that determination of the mutation is at the protein level or nucleic acid level (genomic DNA or mRNA) and are accomplished using techniques such as those described herein. In a particular embodiment, said EGFR inhibitor competes with ATP at the EGFR kinase domain. In a particular embodiment the EGFR inhibitor is erlotinib.

Also disclosed is a method of treating a patient having a tumor incorporating a T790M mutant EGFR protein or gene (or treating a tumor incorporating a T790M mutant EGFR protein or gene) comprising co-administering to said patient (or contacting said tumor with) a first compound that binds to and/or inhibits signaling of said T790M mutant EGFR in combination with a second compound that binds to and/or inhibits signaling of wild-type EGFR or EGFR incorporating an activating mutation. In a particular embodiment said activating mutation is one or more of those described in Table 1 (other than T790M). In a particular embodiment said first and second compounds are administered sequentially or concomitantly. In a particular embodiment said second compound is erlotinib.

Also disclosed is a method of screening for compounds that inhibit signaling of a mutant EGFR protein that incorporates a T790M mutation, comprising contacting said mutant EGFR with a test compound in the presence of a phosphorylation substrate and ATP and detecting a change in the amount of phosphorylation of said substrate whereby a reduction of phosphorylation of said substrate compared to a control, or compared to phosphorylation of the substrate in the absence of the test compound, indicates said test compound is an inhibitor of mutant EGFR signaling. In an embodiment, said method is performed in vitro in the presence of a ligand for said mutant EGFR such as EGF or TGF-alpha.

In a particular embodiment the inhibitory activity of a test compound can be determined in vitro by the amount of inhibition of the phosphorylation of an exogenous substrate (e.g. Lys₃ -Gastrin or polyGluTyr (4:1) random copolymer (I. Posner et. al., J. Biol. Chem. 267 (29), 20638-47 (1992)) on tyrosine by epidermal growth factor receptor kinase by a test compound relative to a control. Purified, soluble human T790M mutant EGFR (96 ng) is preincubated in a microfuge tube with EGF (2 µg/ml) in phosphorylation buffer+vanadate (PBV: 50 mM HEPES, pH 7.4; 125 mM NaCl; 24 mM MgCl₂; 100 µM sodium orthovanadate), in a total volume of 10 µl, for 20-30 minutes at room temperature. The test compound, dissolved in dimethylsulfoxide (DMSO), is diluted in PBV, and 10 µl is mixed with the mutant EGFR/EGF mix, and incubated for 10-30 minutes at 30° C. The phosphorylation reaction is initiated by addition of 20 µl ³³ P- ATP/substrate mix (120 µM Lys₃ -Gastrin (sequence in single letter code for amino acids, KKKGPWLEEEEEAYGWLDF - SEQ ID NO: 38), 50 mM Hepes pH 7.4, 40 µM ATP, 2µCi γ-[³³P]-ATP) to the mutant EGFR/EGF mix and incubated for 20 minutes at room temperature. The reaction is stopped by addition of 10 µl stop solution (0.5M EDTA, pH 8; 2mM ATP) and 6 µl 2N HCl. The tubes are centrifuged at 14,000 RPM, 4°C., for 10 minutes. 35 µl of supernatant from each tube is pipetted onto a 2.5 cm circle of Whatman P81 paper, bulk washed four times in 5% acetic acid, 1 liter per wash, and then air dried. This results in the binding of substrate to the paper with loss of free ATP on washing. The [³³P] incorporated is measured by liquid scintillation counting. Incorporation in the absence of substrate (e.g., lys₃ -gastrin) is subtracted from all values as a background and percent inhibition is calculated relative to controls without test compound present. Such assays, carried out with a range of doses of test compounds, allow the determination of an approximate IC₅₀ value for the in vitro inhibition of T790M mutant EGFR kinase activity.

Also disclosed is a method for identifying a tumor in a human subject that is susceptible to treatment comprising determining the presence of a mutated EGFR gene or mutated EGFR protein in a sample of said tumor wherein said mutation is located in exons 18-21 of EGFR whereby the presence of a mutated EGFR gene or mutated EGFR protein indicates that the tumor is susceptible to treatment with an anticancer agent. In a particular embodiment the anticancer agent is a chemotherapeutic agent which may be a cytotoxic or cytostatic. Tumors include neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyo sarcoma, craniopharyngeoma, osteosarcoma, chondrosarcoma, myosarcoma, liposarcoma, fibrosarcoma, Ewing sarcoma and plasmocytoma. Particular tumors include those of the brain, liver, kidney, bladder, breast, gastric, ovarian, colorectal, prostate, pancreatic, breast, lung, vulval, thyroid, colorectal, oesophageal, hepatic carcinomas, sarcomas, glioblastomas, head and neck, leukemias and lymphoid malignancies.

Particular chemotherapeutic agents include, but are not limited to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphtheria toxin; (Vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate; ET-18- OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists. In an embodiment, the chemotherapeutic compound is one or more of gemcitabine, cisplatin, doxorubicin, daunarubicin, paclitexel, taxotere and mitomycin C. In a particular embodiment the chemotherapeutic compound is one or more of gemcitabine, cisplatin and paclitaxel. In accordance with the invention, the EGFR inhibitor is a small molecule that competes with ATP such as Tarceva^{™} (erlotinib, OSI Pharmaceuticals), Iressa^{™} (gefitinib, Astra-Zeneca), tyrphostins described by Dvir, et al., J Cell Biol., 113:857-865 (1991); tricyclic pyrimidine compounds disclosed in U.S. Patent 5,679,683; compound 6-(2,6- dichlorophenyl)-2-(4-(2-diethylaininoethoxy)phenylamino)-8-methyl-8H-pyrido(2,3-d)pyrimidin-7-one (known as PD 166285) disclosed in Panek, et al., Journal of Pharmacology and Experimental Therapeutics 283, 1433-1444 (1997).

Also disclosed is a method of identifying an EGFR mutation in a sample comprising contacting nucleic acid from said sample with a nucleic acid probe that is capable of specifically hybridizing to nucleic acid encoding a mutated EGFR protein, or fragment thereof incorporating a mutation, and detecting said hybridization. In a particular embodiment said probe is detectably labeled such as with a radioisotope (³H, ³²P, ³³P etc), a fluorescent agent (rhodamine, fluorescene etc.) - or a chromogenic agent. In a particular embodiment the probe is an antisense oligomer, for example PNA, morpholino-phosphoramidates, LNA or 2'-alkoxyalkoxy. The probe may be from about 8 nucleotides to about 100 nucleotides, or about 10 to about 75, or about 15 to about 50, or about 20 to about 30. In another aspect said probes are provided in a kit for identifying EGFR mutations in a sample, said kit comprising an oligonucleotide that specifically hybridizes to or adjacent to a site of mutation in the EGFR gene. The kit may further comprise instructions for treating patients having tumors that contain EGFR mutations with an EGFR inhibitor based on the result of a hybridization test using the kit.

Also disclosed is a method of detecting a mutated EGFR gene in a sample comprising amplifying from said sample nucleic acid corresponding to the kinase domain of said EGFR gene, or exons 18-21, or a fragment thereof suspected of containing a mutation, and comparing the electrophoretic mobility of the amplified nucleic acid to the electrophoretic mobility of corresponding wild-type EGFR gene or fragment thereof. A difference in the mobility indicates the presence of a mutation in the amplified nucleic acid sequence. Electrophoretic mobility may be determined on polyacrylamide gel.

Alternatively, amplified EGFR gene or fragment nucleic acid may be analyzed for detection of mutations using Enzymatic Mutation Detection (EMD) (Del Tito et al, Clinical Chemistry 44:731-739, 1998). EMD uses the bacteriophage resolvase T₄ endonuclease VII, which scans along double-stranded DNA until it detects and cleaves structural distortions caused by base pair mismatches resulting from point mutations, insertions and deletions. Detection of two short fragments formed by resolvase cleavage, for example by gel eletrophoresis, indicates the presence of a mutation. Benefits of the EMD method are a single protocol to identify point mutations, deletions, and insertions assayed directly from PCR reactions eliminating the need for sample purification, shortening the hybridization time, and increasing the signal-to-noise ratio. Mixed samples containing up to a 20-fold excess of normal DNA and fragments up to 4 kb in size can been assayed. However, EMD scanning does not identify particular base changes that occur in mutation positive samples requiring additional sequencing procedures to identity of the mutation if necessary. CEL I enzyme can be used similarly to resolvase T₄ endonuclease VII as demonstrated in US5869245.

Another simple kit for detecting the EGFR mutations is a reverse hybridization test strip similar to Haemochromatosis StripAssay^{™} (Viennalabs http://www.bamburghmarrsh.com/pdf/4220.pdf) for detection of multiple mutations in HFE, TFR2 and FPN1 genes causing Haemochromatosis. Such an assay is based on sequence specific hybridisation following amplification by PCR. For single mutation assays, a microplate-based detection system may be applied, whereas for multi-mutation assays, teststrips may be used as "macro-arrays". Kits may include ready-to use reagents for sample prep, amplification and mutation detection. Multiplex amplification protocols provide convenience and allow testing of samples with very limited volumes. Using the straightforward StripAssay format, testing for twenty and more mutations may be completed in less than five hours without costly equipment. DNA is isolated from a sample and the EGFR gene (or exons 18-21 or KDR or segments thereof) is amplified in vitro (e.g. PCR) and biotin-labeled, preferably in a single ("multiplex") amplification reaction. The PCR products are the selectively hybridized to oligonucleotide probes (wild-type and mutant specific) immobilized on a solid support such as a test strip in which the probes are immobilized as parallel lines or bands. Bound biotinylated amplicons are detected using streptavidin-alkaline phosphatase and color substrates. Such an assay can detect all or any subset of the mutations in table 1. With respect to a particular mutant probe band one of three signaling patterns are possible: (i) a band only for wild-type probe which indicates normal EGFR (ii) bands for both wild-type and a mutant probe which indicates heterozygous genotype and (iii) band only for the mutant probe which indicates homozygous mutant EGFR genotype. Accordingly there is further provideda method of detecting EGFR mutations comprising isolating nucleic acid from a sample, amplifying the EGFR gene, or fragment thereof (e.g. the KDR or exons 18-21 or smaller) such that the amplified nucleic acid comprises a ligand, contacting the amplified EGFR gene or fragment with a probe which comprises a detectable binding partner to the ligand and the probe is capable of specifically hybridizing to an EGFR mutation, and then detecting the hybridization of said probe to said amplified EGFR gene or fragment. In a particular embodiment the ligand is biotin and the binding partner is comprises avidin or streptavidin. In a particular embodiment the binding partner is steptavidin-alkaline which is detectable with color substrates. In a particular embodiment the probes are immobilized for example on a test strip wherein probes complementary to different mutations are separated from one another. Alternatively, the amplified nucleic acid is labeled with a radioisotope in which case the probe need not comprise a ligand.

The tumor samples were also analyzed for mutations in KRAS (as referred to as p21a). Particular mutations detected in exon 1 are: G12C; G12A; G12D; G12R; G12S; G12V; G13C; G13D which correlated with poor prognosis to chemotherapy as well as chemotherapy with erlotinib therapy. Accordingly, the disclosure further provides a method of identifying patients not responsive to therapy of an EGFR inhibitor such as erlotinib or erlotinib in combination with chemotherapy comprising determining the presence or absence of a KRAS mutation whereby the presence of said mutation indicates a patient will not respond to said therapy. Alternatively, there is provided a method for identifying a tumor in a human subject that is susceptible to treatment with an EGFR inhibitor comprising (i) determining the presence of a wild-type KRAS protein or gene in a sample of said tumor whereby the presence of a wild-type KRAS protein or gene indicates that the tumor is susceptible to treatment with an EGFR inhibitor or (ii) determining the presence of a mutated KRAS protein or gene in a sample of said tumor whereby the absence of a mutated KRAS protein or gene indicates that the tumor is susceptible to treatment with an EGFR inhibitor. In a particular embodiment the KRAS mutation is an activating mutation. In a particular embodiment the mutation is in exon 1 of KRAS. In another embodiment the KRAS mutation is at least one of G12C; G12A; G12D; G12R; G12S; G12V; G13C; G13D. Alternatively, individuals who have tumors which harbor mutant KRAS may be treated with EGFR inhibitors when also treated with a KRAS inhibitor either before, after or during treatment with the EGFR inhibitor. Methods for determining the presence of KRAS mutations are analogous to those used to identify EGFR mutations described in detail herein.

It was further observed that patients whose tumors were found to have present KRAS mutations and stained positively for EGFR by IHC had significantly shorter survival when treated with erlotinib in combination with chemotherapy compared to those patients treated with chemotherapy alone. The following tables 3 and 4 summarize these results. Accordingly, the invention provides a method, as defined in the claims, of identifying a patient nonresponsive to treatment of an EGFR inhibitor such as erlotinib in combination with a chemotherapeutic agent, comprising determining the presence or absence of a KRAS mutation and an EGFR in a tumor of said patient whereby the presence of both a KRAS mutation and an EGFR in said tumor indicates a patient will not respond to said EGFR inhibitor therapy in combination with chemotherapy. In this context "nonresponsive" means that a patient will have a reduced survival than a similar patient (having KRAS mutations and the presence of EGFR a in tumor) treated with chemotherapy alone. Said EGFR may be either mutant or wildtype EGFR.
KRAS mutations refers to mutations in the KRAS protein or nucleic acid and is detected using procedures analogous to those for detecting EGFR mutations.
The presence of EGFR is determined by immunohistochemistry (IHC).
In an embodiment, the EGFR is wildtype EGFR. In another embodiment, the EGFR is a mutant EGFR.

Also disclosed is a method of determining whether a tumor will respond to treatment with an EGFR inhibitor in combination with a chemotherapeutic agent, comprising determining in a sample of said tumor the presence of a mutant KRAS protein or nucleic acid and an EGFR, whereby the presence of both a mutant KRAS protein or nucleic acid and an EGFR indicates that the tumor will not respond to treatment with an EGFR inhibitor. In this context, "respond" means that the tumor will shrink in size or volume or the rate of increase in size or volume is reduced. In a particular embodiment, said treatment with an EGFR inhibitor is prior to, concurrent with, or subsequent to with treatment with chemotherapy.

**Table 3**

| **Kras Wild Type** | | | |
|---|---|---|---|
| | erlotinib + Chemo | | Chemo Alone |
| EGFR IHC-n | 48 | | 56 |
| Median OS (mo) (95%Cl) | 12.1 (9.0, 16.6) | | 12.7 (9.1, 16.8) |
| Logrank P-value | | 0.9557 | |
| EGFR IHC+n | 53 | | 38 |
| Median OS (mo) (95%Cl) | 12.1 (8.0, 16.6) | | 10.3 (8.3,.) |
| Logrank P-value | | 0.7892 | |
| Hazard Ratio (95%Cl) | | 1.1 (0.6, 1.9) | |

**Table 4**

| **Kras Mutants** | | | |
|---|---|---|---|
| | erlotinib + Chemo | | Chemo Alone |
| EGFR IHC-n | 11 | | 9 |
| Median OS (mo) (95%Cl) | 9.0 (3.4,12.9) | | 12.8 (3.3,.) |
| Logrank P-value | | 0.5507 | |
| EGFR IHC+n | 12 | | 20 |
| Median OS (mo) (95%Cl) | 3.4 (2.1, 4.4) | | 13.5(11.1,15.1) |
| Logrank P-value | | < 0.001 | |
| Hazard Ratio (95%Cl) | | 4.9 (2.1, 11.5) | |

Alterations of a wild-type gene according to the present invention encompasses all forms of mutations such as insertions, inversions, deletions, and/or point mutations. Somatic mutations are those which occur only in certain tissues, e.g., in the tumor tissue, and are not inherited in the germ line. Germ line mutations can be found in any of a body's tissues. If only a single allele is somatically mutated, an early neoplastic state is indicated. However, if both alleles are mutated then a late neoplastic state is indicated.

According to the diagnostic and prognostic method of the present disclosure, alteration of the wild-type EGFR gene is detected. The finding of EGFR mutations is therefore a diagnostic and prognostic indicator as described herein.

The EGFR mutations found in tumor tissues may result in increased signaling activity relative to wild-type EGFR leading to a cancerous state. In order to detect the alteration of the wild-type EGFR gene a sample or biopsy of the tumor is obtained by methods well known in the art and appropriate for the particular type and location of the tumor. For instance, samples of lung cancer lesions may be obtained by resection, bronchoscopy, fine needle aspiration, bronchial brushings, or from sputum, pleural fluid or blood. Means for enriching a tissue preparation for tumor cells are known in the art. For example, the tissue may be isolated from paraffin or cryostat sections. Cancer cells may also be separated from normal cells by flow cytometry or laser capture microdissection. These as well as other techniques for separating tumor from normal cells are well known in the art. If the tumor tissue is highly contaminated with normal cells, detection of mutations is more difficult.

Detection of point mutations may be accomplished by molecular cloning of the EGFR allele (or alleles) and sequencing that allele(s) using techniques well known in the art. Alternatively, the polymerase chain reaction (PCR) can be used to amplify gene sequences directly from a genomic DNA preparation from the tumor tissue. The DNA sequence of the amplified sequences can then be determined and mutations identified therefrom. The polymerase chain reaction is well known in the art and described in Saiki et al., Science 239:487, 1988; U.S. 4,683,203; and U.S. 4,683,195.

Specific primer pairs which can be used for PCR amplification of EGFR exons 18-21 include:
<5pEGFR.ex18.out> CAAATGAGCTGGCAAGTGCCGTGTC (SEQ ID NO: 39)
<3pEGFR.ex18.out> GAGTTTCCCAAACACTCAGTGAAAC (SEQ ID NO: 40)
<5pEGFR.ex19.out> GCAATATCAGCCTTAGGTGCGGCTC (SEQ ID NO: 41)
<3pEGFR.ex19.out> CATAGAAAGTGAACATTTAGGATGTG (SEQ ID NO: 42)
<5pEGFR.ex20.out> CCATGAGTACGTATTTTGAAACTC (SEQ ID NO: 43)
<3pEGFR.ex20.out> CATATCCCCATGGCAAACTCTTGC (SEQ ID NO: 44)
<5pEGFR.ex21.out> CTAACGTTCGCCAGCCATAAGTCC (SEQ ID NO: 45)
<3pEGFR.ex21.out> GCTGCGAGCTCACCCAGAATGTCTGG (SEQ ID NO: 46)
<5pEGFR.ex18.in.m13f> TGTAAAACGACGGCCAGTCAAGTGCCGTGTCCTGGCACCCAAGC (SEQ ID NO: 47)
<3pEGFR.ex18.in.m13r> CAGGAAACAGCTATGACCCCAAACACTCAGTGAAACAAAGAG (SEQ ID NO: 48)
<5pEGFR.ex19.in.m13f> TGTAAAACGACGGCCAGTCCTTAGGTGCGGCTCCACAGC (SEQ ID NO: 49)
<3pEGFR.ex1.9.in.m13r> CAGGAAACAGCTATGACCCATTTAGGATGTGGAGATGAGC (SEQ ID NO: 50)
<5pEGFR.ex20.in.m13f> TGTAAAACGACGGCCAGTGAAACTCAAGATCGCATTCATGC (SEQ ID NO: 51)
<3pEGFR.ex20.in.m13r> CAGGAAACAGCTATGACCGCAAACTCTTGCTATCCCAGGAG (SEQ ID NO: 52)
<5pEGFR.ex21.in.m13f> TGTAAAACGACGGCCAGTCAGCCATAAGTCCTCGACGTGG (SEQ ID NO: 53)
<3pEGFR.ex21.in.m13r> CAGGAAACAGCTATGACCCATCCTCCCCTGCATGTGTTAAAC (SEQ ID NO: 54)
Specific primer pairs which can be used for PCR amplification of K-Ras exon 1 include:
<5pKRAS-out> TACTGGTGGAGTATTTGATAGTG (SEQ ID NO: 55)
<3pKRAS-out> CTGTATCAAAGAATGGTCCTG (SEQ ID NO: 56)
<5pKRAS-in.m13f> TGTAAAACGACGGCCAGTTAGTGTATTAACCTTATGTG (SEQ ID NO: 57)
<3pKRAS-in.m13r> CAGGAAACAGCTATGACCACCTCTATTGTTGGATCATATTCG (SEQ ID NO: 58)

The ligase chain reaction, which is known in the art, can also be used to amplify EGFR sequences. See Wu et al., Genomics, Vol. 4, pp. 560-569 (1989). In addition, a technique known as allele specific PCR can be used. (See Ruano and Kidd, Nucleic Acids Research, Vol. 17, p. 8392, 1989.) According to this technique, primers are used which hybridize at their 3'ends to a particular EGFR mutation. If the particular EGFR mutation is not present, an amplification product is not observed. Amplification Refractory Mutation System (ARMS) can also be used as disclosed in European Patent Application Publication No. 0332435 and in Newton et al., Nucleic Acids Research, Vol. 17, p.7, 1989. Insertions and deletions of genes can also be detected by cloning, sequencing and amplification. In addition, restriction fragment length polymorphism, (RFLP) probes for the gene or surrounding marker genes can be used to score alteration of an allele or an insertion in a polymorphic fragment. Single stranded conformation polymorphism (SSCP) analysis can also be used to detect base change variants of an allele. (Orita et al., Proc. Natl. Acad. Sci. USA Vol. 86, pp. 2766-2770, 1989, and Genomics, Vol. 5, pp. 874-879, 1989). Other techniques for detecting insertions and deletions as are known in the art can be used.

Alteration of wild-type genes can also be detected on the basis of the alteration of a wild-type expression product of the gene. Such expression products include both the EGFR mRNA as well as the EGFR protein product. Point mutations may be detected by amplifying and sequencing the mRNA or via molecular cloning of cDNA made from the mRNA. The sequence of the cloned CDNA can be determined using DNA sequencing techniques which are well known in the art. The cDNA can also be sequenced via the polymerase chain reaction (PCR).

Mismatches, according to the present invention are hybridized nucleic acid duplexes which are not 100% complementary. The lack of total complementarity may be due to deletions, insertions, inversions, substitutions or frameshift mutations. Mismatch detection can be used to detect point mutations in the gene or its mRNA product. While these techniques are less sensitive than sequencing, they are simpler to perform on a large number of tumor samples. An example of a mismatch cleavage technique is the RNase protection method, which is described in detail in Winter et al., Proc. Natl. Acad. Sci. USA, Vol. 82, p. 7575, 1985 and Meyers et al., Science, Vol. 230, p. 1242, 1985. This method involves the use of a labeled riboprobe which is complementary to the human wild-type EGFR gene coding sequence (or exons 18-21 or KDR thereof). The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the EGFR mRNA or gene but can be exons 18 through 21 or the EGFR KDR or segments thereof. If the riboprobe comprises only a segment of the EGFR mRNA or gene it will be desirable to use a number of these probes to screen the whole mRNA sequence for mismatches.

In a similar manner, DNA probes can be used to detect mismatches, through enzymatic or chemical cleavage. See, e.g., Cotton et al., Proc. Natl. Acad. Sci. USA, Vol. 85, 4397, 1988; and Shenk et al., Proc. Natl. Acad. Sci. USA, Vol. 72, p. 989, 1975. Alternatively, mismatches can be detected by shifts in the electrophoretic mobility of mismatched duplexes relative to matched duplexes. See, e.g., Cariello, Human Genetics, Vol. 42, p. 726, 1988. With either riboprobes or DNA probes, the cellular mRNA or DNA which might contain a mutation can be amplified using PCR before hybridization. Changes in DNA of the EGFR gene can also be detected using Southern hybridization, especially if the changes are gross rearrangements, such as deletions and insertions.

DNA sequences of the EGFR gene which have been amplified by use of polymerase chain reaction may also be screened using allele-specific probes. These probes are nucleic acid oligomers, each of which contains a region of the EGFR gene sequence harboring a known mutation. For example, one oligomer may be about 30 nucleotides in length, corresponding to a portion of the EGFR gene sequence. By use of a battery of such allele-specific probes, PCR amplification products can be screened to identify the presence of a previously identified mutation in the EGFR gene. Hybridization of allele-specific probes with amplified EGFR sequences can be performed, for example, on a nylon filter. Hybridization to a particular probe under stringent hybridization conditions indicates the presence of the same mutation in the tumor tissue as in the allele-specific probe.

DNA sequences of the EGFR gene which have been amplified by use of polymerase chain reaction may also be screened for mutations by mass spectroscopy techniques. Amplified regions of the gene having a mutation will have a different mass spec signature than the same region without mutations.

Alteration of wild-type EGFR genes can also be detected by screening for alteration of wild-type EGFR protein. For example, monoclonal antibodies immunoreactive with EGFR can be used to screen a tissue. Lack of cognate antigen would indicate an EGFR mutation. Antibodies specific for products of mutant alleles could also be used to detect mutant EGFR gene product. Antibodies may be identified from phage display libraries. Such immunological assays can be done in any convenient format known in the art. These include Western blots, immunohistochemical assays and ELISA assays. Any means for detecting an altered EGFR protein can be used to detect alteration of wild-type EGFR genes.

Mutant EGFR genes or gene products can be detected from tumor or from other body samples such as urine, sputum or serum. The same techniques discussed above for detection of mutant EGFR genes or gene products in tumor samples can be applied to other body samples. Cancer cells are sloughed off from tumors and appear in such body samples. By screening such body samples, a simple early diagnosis can be achieved for many types of cancers. In addition, the progress of chemotherapy or radiotherapy can be monitored more easily by testing such body samples for mutant EGFR genes or gene products.

The methods of diagnosis of the present disclosure are applicable to any tumor in which EGFR has a role in tumorigenesis for example lung, breast, colon, glioma, bladder, liver, stomach and prostate. The diagnostic method of the present disclosure is useful for clinicians so that they can decide upon an appropriate course of treatment. For example, a tumor displaying alteration of both EGFR alleles might suggest a more aggressive therapeutic regimen than a tumor displaying alteration of only one EGFR allele.

The primer pairs of the present disclosure are useful for determination of the nucleotide sequence of a particular EGFR allele using the polymerase chain reaction. The pairs of single stranded DNA primers can be annealed to sequences within or surrounding the EGFR gene on in order to prime amplifying DNA synthesis of the EGFR gene itself. A set of these primers allows synthesis of all of the nucleotides of the EGFR exons 18 through 21. Allele specific primers can also be used. Such primers anneal only to particular EGFR mutant alleles, and thus will only amplify a product in the presence of the mutant allele as a template. In order to facilitate subsequent cloning of amplified sequences, primers may have restriction enzyme site sequences appended to their ends. Thus, all nucleotides of the primers are derived from EGFR exons 18-21 or sequences adjacent thereto except the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Design of particular primers is well within the skill of the art.

The nucleic acid probes provided by the present disclosure are useful for a number of purposes. They can be used in Southern hybridization to genomic DNA and in the RNase protection method for detecting point mutations already discussed above. The probes can be used to detect PCR amplification products. They may also be used to detect mismatches with the EGFR gene or mRNA using other techniques. Mismatches can be detected using either enzymes (e.g., S1 nuclease), chemicals (e.g., hydroxylamine or osmium tetroxide and piperidine), or changes in electrophoretic mobility of mismatched hybrids as compared to totally matched hybrids. These techniques are known in the art. See Novack et al., Proc. Natl. Acad. Sci. USA, Vol. 83, p. 586, 1986. Generally, the probes are complementary to EGFR exon 18-21 sequences, although generally probes to the kinase domain and segments thereof are also contemplated. An entire battery of nucleic acid probes may be used to compose a kit for detecting alteration of wild-type EGFR genes. The kit allows for hybridization to the entire exon 18-21 sequence of the EGFR gene. The probes may overlap with each other or be contiguous.

If a riboprobe is used to detect mismatches with mRNA, it is complementary to the mRNA of the EGFR gene. the riboprobe thus is an antisense probe in that it does not code for the EGFR protein because it is complementary to the sense strand. The riboprobe generally will be labeled with a radioactive, colorimetric, or fluorometric material, which can be accomplished by any means known in the art. If the riboprobe is used to detect mismatches with DNA it can be of either polarity, sense or anti-sense. Similarly, DNA probes also may be used to detect mismatches.

Predisposition to cancers can be ascertained by testing any tissue of a human for mutations of the EGFR gene. For example, a person who has inherited a germ line EGFR mutation would be prone to develop cancers. This can be determined by testing DNA from any tissue of the body. For example, blood can be drawn and DNA extracted from the cells of the blood. In addition, prenatal diagnosis can be accomplished by testing fetal cells, placental cells, or amniotic fluid for mutations of the EGFR gene. Alteration of a wild- type EGFR allele, whether for example, by point mutation or by deletion, can be detected by any of the means discussed above.

### EXAMPLES:

### Example 1 Slide Preparation - Deparaffinization and Staining

Submersed sections in the following solutions:
Fresh xylenes (to depariffinize the sections) - 5 min
Fresh xylenes - 5 min
100% ethanol - 15 sec
95% ethanol - 15 sec
70% ethanol - 15 sec
Deionized water - 15 sec
Mayer's Hematoxylin - 30 sec
Deionized water - rinse (x 2) - 15 sec
70% ethanol - 15 sec
Eosin Y - 5 sec
95% ethanol - 15 sec
95% ethanol - 15 sec
100% ethanol - 15 sec
100% ethanol - 15 sec
Xylenes (to ensure dehydration of the section) - 60 sec
Air-dried for approximately 2 minutes or gently used air gun to completely remove xylenes.
The tissue was then ready for LCM.

### Example 2 Laser Capture Microdissection and DNA Extraction

### Materials:

PixCell II LCM System
CapSure HS or CapSure Macro LCM caps
ExtractSure device (HS only)
Razor blades (factory sterile)
0.5 ml tubes
0.2 ml tubes
PicoPure DNA extraction Kit
65°C incubator

### Procedure:

Placed CapSure cap over area of tissue to be collected
2. Lased over desired area
Lifted cap off tissue.
Dispensed 20 ul of PicoPure digest buffer with Proteinase K into 0.5ml tube.
Placed cap with dissected material into tube to form a tight seal.
Inverted tube such that digest buffer covered cap.
Incubated at 65°C for 24 hours.
Spun tube with cap to collect digested material in the bottom of the tube.
Transferred digest to 0.2 ml strip tube.
Inactivated Proteinase K at 95°C for 10 minutes in a thermocycler with a heated lid.
10. Used 1-2 ul of sample in a 50 ul PCR reaction. No clean-up was necessary.

### Example 3 PCR amplification

### PCR Primers:

Primer pairs were designed for each exon to be sequenced (*EGFR* exons 18, 19, 20 and 21). Primer sequences used were as follows:
<5pEGFR.ex18.out> CAAATGAGCTGGCAAGTGCCGTGTC (SEQ ID NO: 39)
<3pEGFR.ex18.out> GAGTTTCCCAAACACTCAGTGAAAC (SEQ ID NO: 40)
<5pEGFR.ex19.out> GCAATATCAGCCTTAGGTGCGGCTC (SEQ ID NO: 41)
<3pEGFR.ex19.out> CATAGAAAGTGAACATTTAGGATGTG (SEQ ID NO: 42)
<5pEGFR.ex20.out> CCATGAGTACGTATTTTGAAACTC (SEQ ID NO: 43)
<3pEGFR.ex20.out> CATATCCCCATGGCAAACTCTTGC (SEQ ID NO: 44)
<5pEGFR.ex21.out> CTAACGTTCGCCAGCCATAAGTCC (SEQ ID NO: 45)-<3pEGFR.ex21.out> GCTGCGAGCTCACCCAGAATGTCTGG (SEQ ID NO: 46)
<5pEGFR.ex18.in.m13f> TGTAAAACGACGGCCAGTCAAGTGCCGTGTCCTGGCACCCAAGC (SEQ ID NO: 47)
<3pEGFR.ex18.in.m13r> CAGGAAACAGCTATGACCCCAAACACTCAGTGAAACAAAGAG (SEQ ID NO: 48)
<5pEGFR.ex19.in.m13f> TGTAAAACGACGGCCAGTCCTTAGGTGCGGCTCCACAGC (SEQ ID NO: 49)
<3pEGFR.ex19.in.m13r> CAGGAAACAGCTATGACCCATTTAGGATGTGGAGATGAGC (SEQ ID NO: 50)
<5pEGFR.ex20.in.m13f> TGTAAAACGACGGCCAGTGAAACTCAAGATCGCATTCATGC (SEQ ID NO: 51)
<3pEGFR:ex20.in.m13r> CAGGAAACAGCTATGACCGCAAACTCTTGCTATCCCAGGAG (SEQ ID NO: 52)
<5pEGFR.ex21.in.m13f> TGTAAAACGACGGCCAGTCAGCCATAAGTCCTCGACGTGG (SEQ ID NO: 53)
<3pEGFR.ex21.in.m13r> CAGGAAACAGCTATGACCCATCCTCCCCTGCATGTGTTAAAC (SEQ ID NO: 54)

### K-Ras oligos for PCR

<5pKRAS-out> TACTGGTGGAGTATTTGATAGTG (SEQ ID NO: 55)
<3pKRAS-out> CTGTATCAAAGAATGGTCCTG (SEQ ID NO: 56)
<5pKRAS-in.m13f> TGTAAAACGACGGCCAGTTAGTGTATTAACCTTATGTG (SEQ ID NO: 57)
<3pKRAS-in>m13r CAGGAAACAGCTATGACCACCTCTATTGTTGGATCATATTCG (SEQ ID NO: 58)

Nested amplification of the primary PCR product was performed using intron-specific primer pairs located within the primary PCR product. These nested primers pairs were tagged with M13f and M13rev sequences.

### First round of PCR:

### PCR Reaction:

| | |
|---|---|
| DNA | 0.5 to 30ng |
| Primers | 250nM/ each outer primers |
| dNTPs | 0.2mM each (Roche cat#1581295) |
| MgCl₂ | 1.5mM (15mM 10 X buffer) |
| Enzyme | 1.5 U/ RX Expand Hiqh fidelity Taq (Roche cat#1759078) |
| | 50ul reaction volume |

### Thermocycler conditions:

95°C - 3 minutes
94°C - 30 seconds repeat 35 times
58°C - 30 seconds
72°C - 1 minute
72°C - 8 minutes
4°C - forever

### Second round of PCR:

### PCR Reaction:

| | |
|---|---|
| DNA | 1 ul from first round PCR reaction |
| Primers | 250nM/ each inner primers |
| dNTPs | 0.2mM each (Roche cat#1581295) |
| MgCl₂ | 1.5mM (15mM 10 X buffer) |
| Enzyme | 1.5 U/ RX Expand Hiqh fidelity Taq (Roche cat#1759078) |
| | 50ul reaction volume |

### Thermocycler conditions:

95°C - 3 minutes
94°C - 30 seconds repeat 30 times
58°C - 30 seconds
72°C - 1 minute
72°C - 8 minutes
4°C - forever

### Isolation of PCR Products:

PCR reaction products were run on E-Gel 2% agarose gels (Invitrogen, cat# G6018-02) for quality control. PCR products were purified directly using the Qiaquick 96 PCR purification kit (Qiagen, cat#28181) or gel purified as was necessary. For gel purification, the PCR product was excised from the E-gel and the DNA purified using Qiaquick 96 PCR purification kit with a gel extraction protocol (Qiagen, cat#28181).

### Example 4 Sequencing

Nested sequencing primers or standard M13f and M13rev sequencing primers for tagged PCR products were used to sequence the purified PCR products. Sequences were as follows:
<m13f> TGTAAAACGACGGCCAGT (SEQ ID NO: 59)
<m13r> CAGGAAACAGCTATGACC (SEQ ID NO: 60)

Purified PCR products were diluted and cycle-sequenced using the BigDye Terminator Kit (ABI, Foster City, CA) according to manufacturer's instructions.

### Reaction Mix:

5 ul DNA (25-100ng PCR product)
6 ul water
1 ul primer diluted to .25 OD/100ul with water (m13f or m13r or sequence specific primer)
2 ul BigDye v3.1
6 ul Dilution Buffer (equivalent of ABI 5x Dilution Buffer)

### Cycle Sequencing:

### Conditions:

96°C - 2.5 minutes - initial denaturation
96°C - 10 seconds
50°C - 5 seconds
60°C - 4 minutes
repeated for 25 to 50 total cycles

### Reaction Cleanup:

Removed unincorporated nucleotides using:
8% sephadex
500 ul in Edge BioSystem 96-well block
spin @ 750g for 2 minutes

### Analysis:

Reaction products were electrophoresed on ABI3700 or ABI3730 sequencing instruments.

Electropherograms were analyzed for mutations using commercially available analysis programs, such as Sequencher (Gene Codes, Corp), and with custom tools.

### Example 5 Dose Response

Human epidermal growth factor receptor (EGFR) wild-type and mutant constructs used in this study were epitope-tagged at the N-terminus with the herpes simplex virus signal sequence of gD, replacing the endogenous EGFR signal sequence (Schaefer et al. 1999 J. Biol. Chem. 274, 859-866). Cos7 cells were seeded in 12 well dishes in normal growth medium 24 hours prior to transfection. Cells were transfected with 0.25ug per well with expression plasmid DNAs (pRK5.gD.EGFR wild-type, pRK5.gD.EGFR. L858R, or pRK5.gD.EGFR.del(E746-S752) using LipofectAMINE 2000 following manufacturer's recommended protocol (Invitrogen). Twenty-four hours post-transfection, cells were serum starved for six hours in serum free DMEM. One hour prior to stimulation, transfected cells were preincubated with the indicated concentrations of erlotinib. Transfected cells were stimulated with 1 nM TGFα for 10 minutes. Cells were lysed directly in the wells using reducing Laemmli buffer. Receptor autophosphorylation, an index of EGFR receptor activation by growth factor stimulation, was detected by Western blotting using an HRP-conjugated anti-phosphotyrosine antibody (Oncogene Sciences, AB-4). Transfection efficiency was evaluated using an antibody specific for the gD epitope tag (5B6). Level of receptor activation was evaluated from the autoradiograms using NIH Image software. These data were then used to generate a graph from which an IC50 was calculated using a 4 parameter fit function. As illustrated by the results below, erlotinib has a greater affinity to EGFR containing mutations compared to wild-type EGFR.

| EGFR construct | inhibition (IC50) |
|---|---|
| WT EGFR-gD | 50 nM |
| L858R EGFR-gD | 20 nM |
| del(746-752) EGFR-gD | 5 nM |

## Claims

1. A method of identifying a patient expected to have shorter survival if treated with an EGFR inhibitor in combination with chemotherapy, than if treated with chemotherapy alone, comprising determining in vitro the presence or absence of a mutation in the KRAS protein and wildtype or mutated EGFR in a tumor of said patient whereby the presence of both a mutation in the KRAS protein and EGFR in said tumor indicates a patient is expected to have shorter survival if treated with an EGFR inhibitor in combination with chemotherapy, than if treated with chemotherapy alone, wherein the tumour is a lung tumor, wherein the EGFR inhibitor is a small molecule that competes with ATP, and wherein the presence of EGFR is determined by immunohistochemistry.

2. The method of claim 1 wherein said KRAS mutation is an activating mutation.

3. The method of claim 1 wherein said KRAS mutation is at least one of G12C; G12A; G12D; G12R; G12S; G12V; G13C; and G13D.

4. The method of any preceding claim wherein said EGFR inhibitor is one or more of erlotinib or gefitinib.

5. The method of claim 4 wherein said EGFR inhibitor is erlotinib.

6. The method of any preceding claim wherein said chemotherapeutic agent is one or more of cytarabine, fludarabine, 5-fluoro-2'-deoxyuiridine, gemcitabine, hydroxyurea, methotrexate, bleomycin, chlorambucil, cisplatin, cyclophosphamide, doxorubicin, mitoxantrone, camptothecin, topotecan, teniposide, colcemid, colchicine, paclitaxel, vinblastine, vincristine or tamoxifen.

7. The method of any one of claims 1 to 5 wherein said chemotherapeutic agent is carboplatin and/or paclitaxel.

8. The method of any preceding claim wherein the EGFR is wildtype EGFR.

9. The method of any one of claims 1 to 7 wherein the EGFR is mutated EGFR.

10. The method of any preceding claim wherein the presence of a mutation in the KRAS protein is determined by amplifying KRAS nucleic acid from said tumor, or a fragment thereof suspected of containing a mutation, and sequencing said amplified nucleic acid.

11. The method of any one of claims 1 to 9 wherein the presence of a mutation in the KRAS protein is determined by amplifying KRAS nucleic acid from said tumor, or a fragment thereof suspected of containing a mutation, and comparing the electrophoretic mobility of the amplified nucleic acid to the electrophoretic mobility of corresponding wild-type KRAS nucleic acid or fragment.

## Patentansprüche

1. Verfahren zur Identifikation eines Patienten, von dem erwartet wird, dass seine Überlebensdauer kürzer ist, wenn er mit einem EGFR-Inhibitor in Kombination mit Chemotherapie behandelt wird, als wenn er nur mit Chemotherapie behandelt wird, welches das In-vitro-Bestimmen der Gegenwart oder Abwesenheit einer Mutation des KRAS-Proteins und eines Wildtyp- oder mutierten EGFR in einem Tumor von dem Patienten umfasst, worin die Gegenwart sowohl einer Mutation des KRAS-Proteins als auch von EGFR in dem Tumor anzeigt, dass erwartet wird, dass die Überlebensdauer des Patienten kürzer ist, wenn er mit einem EGFR-Inhibitor in Kombination mit Chemotherapie behandelt wird, als wenn er nur mit Chemotherapie behandelt wird, worin der Tumor ein Lungentumor ist, worin der EGFR-Inhibitor ein kleines Molekül ist, das mit ATP konkurriert, und worin die Gegenwart von EGFR durch Immunhistochemie bestimmt wird.

2. Verfahren nach Anspruch 1, worin die KRAS-Mutation eine aktivierende Mutation ist.

3. Verfahren nach Anspruch 1, worin die KRAS-Mutation zumindest eine aus G12C, G12A, G12D, G12R, G12S, G12V, G13C und G13D ist.

4. Verfahren nach einem der vorangegangenen Ansprüche, worin der EGFR-Inhibitor eines oder mehrere aus Erlotinib und Gefitinib ist.

5. Verfahren nach Anspruch 4, worin der EGFR-Inhibitor Erlotinib ist.

6. Verfahren nach einem der vorangegangenen Ansprüche, worin das Chemotherapeutikum eines oder mehrere aus Cytarabin, Fludarabin, 5-Fluor-2'-desoxyuridin, Gemcitabin, Hydroxyharnstoff, Methotrexat, Bleomycin, Chlorambucil, Cisplatin, Cyclophosphamid, Doxorubicin, Mitoxantron, Camptothecin, Topotecan, Teniposid, Colcemid, Colchicin, Paclitaxel, Vinblastin, Vincristin und Tamoxifen ist.

7. Verfahren nach einem der Ansprüche 1 bis 5, worin das Chemotherapeutikum Carboplatin und/oder Paclitaxel ist.

8. Verfahren nach einem der vorangegangenen Ansprüche, worin der EGFR ein Wildtyp-EGFR ist.

9. Verfahren nach einem der Ansprüche 1 bis 7, worin der EGFR ein mutierter EGFR ist.

10. Verfahren nach einem der vorangegangenen Ansprüche, worin die Gegenwart einer Mutation des KRAS-Proteins durch Amplifizieren von KRAS-Nucleinsäure aus dem Tumor oder eines Fragments davon, von der/dem vermutet wird, dass sie/es eine Mutation enthält, und Sequenzieren der amplifizierten Nucleinsäure bestimmt wird.

11. Verfahren nach einem der Ansprüche 1 bis 9, worin die Gegenwart einer Mutation des KRAS-Proteins durch Amplifizieren einer KRAS-Nucleinsäure aus dem Tumor oder eines Fragments davon, von der/dem vermutet wird, dass sie/es eine Mutation enthält, und Vergleichen der elektrophoretischen Mobilität der amplifizierten Nucleinsäure mit der elektrophoretischen Mobilität der entsprechenden Wildtyp-KRAS-Nucleinsäure oder eines Fragments davon bestimmt wird.

## Revendications

1. Procédé pour identifier un patient pour lequel est prévue une survie réduite s'il est traité avec un inhibiteur de EGFR en association avec la chimiothérapie, par rapport à son traitement par chimiothérapie seulement, comprenant la détermination in vitro de la présence ou de l'absence d'une mutation de la protéine KRAS et du EGFR de type sauvage ou muté dans une tumeur dudit patient, la présence à la fois d'une mutation dans la protéine KRAS et de EGFR dans ladite tumeur indiquant ainsi qu'un patient devrait avoir une survie réduite s'il est traité avec un inhibiteur de EGFR en association avec la chimiothérapie, par rapport à son traitement par chimiothérapie seulement, ladite tumeur étant une tumeur du poumon, ledit inhibiteur de EGFR étant une petite molécule qui entre en compétition avec l'ATP, et la présence de EGFR étant déterminée par immunohistochimie.

2. Procédé suivant la revendication 1, dans lequel ladite mutation KRAS est une mutation activatrice.

3. Procédé suivant la revendication 1, dans lequel ladite mutation KRAS est au moins une de G12C ; G12A ; G12D ; G12R ; G12S ; G12V ; G13C ; et G13D.

4. Procédé suivant l'une quelconque des revendications précédentes, dans lequel ledit inhibiteur de EGFR est un ou plusieurs de l'erlotinib et du géfitinib.

5. Procédé suivant la revendication 4, dans lequel ledit inhibiteur de EGFR est l'erlotinib.

6. Procédé suivant l'une quelconque des revendications précédentes, dans lequel ledit agent chimiothérapeutique est un ou plusieurs de la cytarabine, de la fludarabine, de la 5-fluoro-2'-désoxyuridine, de la gemcitabine, de l'hydroxy-urée, du méthotrexate, de la bléomycine, du chlorambucil, du cisplatine, du cyclophosphamide, de la doxorubicine, de la mitoxantrone, de la camptothécine, du topotécan, du téniposide, du colcémide, de la colchicine, du paclitaxel, de la vinblastine, de la vincristine et du tamoxifène.

7. Procédé suivant l'une quelconque des revendications 1 à 5, dans lequel ledit agent chimiothérapeutique est le carboplatine et/ou le paclitaxel.

8. Procédé suivant l'une quelconque des revendications précédentes, dans lequel le EGFR est le EGFR de type sauvage.

9. Procédé suivant l'une quelconque des revendications 1 à 7, dans lequel le EGFR est un EGFR muté.

10. Procédé suivant l'une quelconque des revendications précédentes, dans lequel la présence d'une mutation dans la protéine KRAS est déterminée par amplification de l'acide nucléique KRAS provenant de ladite tumeur, ou d'un de ses fragments supposés contenir une mutation, et par séquençage dudit acide nucléique amplifié.

11. Procédé suivant l'une quelconque des revendications 1 à 9, dans lequel la présence d'une mutation dans la protéine KRAS est déterminée par amplification de l'acide nucléique KRAS provenant de ladite tumeur, ou d'un de ses fragments supposés contenir une mutation, et par comparaison de la mobilité électrophorétique de l'acide nucléique amplifié avec la mobilité électrophorétique de l'acide nucléique KRAS de type sauvage correspondant ou du fragment correspondant.


### Sequence Listing

<110> GENENTECH, INC. KENNETH J. HILLAN
<120> EGFR and KRAS Mutations
<130> 39766-0178PC
<140> TO BE ASSIGNED
   <141> 2006-06-13
<150> US 60/695, 174
   <151) 2006-6-28
<160> 60
<210> 1
   <211> 1210
   <212> PRT
   <213> Homo sapiens
<400> 1
<210> 2
   <211> 5616
   <212> DNA
   <213> Homo sapiens
<400> 2
   ccccggcgca gcgcggccgc agcagcctcc gccccccgca cggtgtgagc 50
   gcccgacgcg gccgaggcgg ccggagtccc gagctagccc cggcggccgc 100
   cgccgcccag accggacgac aggccacctc gtcggcgtcc gcccgagtcc 150
   ccgcctcgcc gccaacgcca caaccaccgc gcacggcccc ctgactccgt 200
   ccagtattga tcgggagagc cggagcgagc tcttcgggga gcagcgatgc 250
   gaccctccgg gacggccggg gcagcgctcc tggcgctgct ggctgcgctc 300
   tgcccggcga gtcgggctct ggaggaaaag aaagtttgcc aaggcacgag 350
   taacaagctc acgcagttgg gcacttttga agatcatttt ctcagcctcc 400
   agaggatgtt caataactgt gaggtggtcc ttgggaattt ggaaattacc 450
   tatgtgcaga ggaattatga tctttccttc ttaaagacca tccaggaggt 500
   ggctggttat gtcctcattg ccctcaacac agtggagcga attcctttgg 550
   aaaacctgca gatcatcaga ggaaatatgt actacgaaaa ttcctatgcc 600
   ttagcagtct tatctaacta tgatgcaaat aaaaccggac tgaaggagct 650
   gcccatgaga aatttacagg aaatcctgca tggcgccgtg cggttcagca 700
   acaaccctgc cctgtgcaac gtggagagca tccagtggcg ggacatagtc 750
   agcagtgact ttctcagcaa catgtcgatg gacttccaga accacctggg 800
   cagctgccaa aagtgtgatc caagctgtcc caatgggagc tgctggggtg 850
   caggagagga gaactgccag aaactgacca aaatcatctg tgcccagcag 900
   tgctccgggc gctgccgtgg caagtccccc agtgactgct gccacaacca 950
   gtgtgctgca ggctgcacag gcccccggga gagcgactgc ctggtctgcc 1000
   gcaaattccg agacgaagcc acgtgcaagg acacctgccc cccactcatg 1050
   ctctacaacc ccaccacgta ccagatggat gtgaaccccg agggcaaata 1100
   cagctttggt gccacctgcg tgaagaagtg tccccgtaat tatgtggtga 1150
   cagatcacgg ctcgtgcgtc cgagcctgtg gggccgacag ctatgagatg 1200
   gaggaagacg gcgtccgcaa gtgtaagaag tgcgaagggc cttgccgcaa 1250
   agtgtgtaac ggaataggta ttggtgaatt taaagactca ctctccataa 1300
   atgctacgaa tattaaacac ttcaaaaact gcacctccat cagtggcgat 1350
   ctccacatcc tgccggtggc atttaggggt gactccttca cacatactcc 1400
   tcctctggat ccacaggaac tggatattct gaaaaccgta aaggaaatca 1450
   cagggttttt gctgattcag gcttggcctg aaaacaggac ggacctccat 1500
   gcctttgaga acctagaaat catacgcggc aggaccaagc aacatggtca 1550
   gttttctctt gcagtcgtca gcctgaacat aacatccttg ggattacgct 1600
   ccctcaagga gataagtgat ggagatgtga taatttcagg aaacaaaaat 1650
   ttgtgctatg caaatacaat aaactggaaa aaactgtttg ggacctccgg 1700
   tcagaaaacc aaaattataa gcaacagagg tgaaaacagc tgcaaggcca 1750
   caggccaggt ctgccatgcc ttgtgctccc ccgagggctg ctggggcccg 1800
   gagcccaggg actgcgtctc ttgccggaat gtcagccgag gcagggaatg 1850
   cgtggacaag tgcaaccttc tggagggtga gccaagggag tttgtggaga 1900
   actctgagtg catacagtgc cacccagagt gcctgcctca ggccatgaac 1950
   atcacctgca caggacgggg accagacaac tgtatccagt gtgcccacta 2000
   cattgacggc ccccactgcg tcaagacctg cccggcagga gtcatgggag 2050
   aaaacaacac cctggtctgg aagtacgcag acgccggcca tgtgtgccac 2100
   ctgtgccatc caaactgcac ctacggatgc actgggccag gtcttgaagg 2150
   ctgtccaacg aatgggccta agatcccgtc catcgccact gggatggtgg 2200
   gggccctcct cttgctgctg gtggtggccc tggggatcgg cctcttcatg 2250
   cgaaggcgcc acatcgttcg gaagcgcacg ctgcggaggc tgctgcagga 2300
   gagggagctt gtggagcctc ttacacccag tggagaagct cccaaccaag 2350
   ctctcttgag gatcttgaag gaaactgaat tcaaaaagat caaagtgctg 2400
   ggctccggtg cgttcggcac ggtgtataag ggactctgga tcccagaagg 2450
   tgagaaagtt aaaattcccg tcgctatcaa ggaattaaga gaagcaacat 2500
   ctccgaaagc caacaaggaa atcctcgatg aagcctacgt gatggccagc 2550
   gtggacaacc cccacgtgtg ccgcctgctg ggcatctgcc tcacctccac 2600
   cgtgcagctc atcacgcagc tcatgccctt cggctgcctc ctggactatg 2650
   tccgggaaca caaagacaat attggctccc agtacctgct caactggtgt 2700
   gtgcagatcg caaagggcat gaactacttg gaggaccgtc gcttggtgca 2750
   ccgcgacctg gcagccagga acgtactggt gaaaacaccg cagcatgtca 2800
   agatcacaga ttttgggctg gccaaactgc tgggtgcgga agagaaagaa 2850
   taccatgcag aaggaggcaa agtgcctatc aagtggatgg cattggaatc 2900
   aattttacac agaatctata cccaccagag tgatgtctgg agctacgggg 2950
   tgaccgtttg ggagttgatg acctttggat ccaagccata tgacggaatc 3000
   cctgccagcg agatctcctc catcctggag aaaggagaac gcctccctca 3050
   gccacccata tgtaccatcg atgtctacat gatcatggtc aagtgctgga 3100
   tgatagacgc agatagtcgc ccaaagttcc gtgagttgat catcgaattc 3150
   tccaaaatgg cccgagaccc ccagcgctac cttgtcattc agggggatga 3200
   aagaatgcat ttgccaagtc ctacagactc caacttctac cgtgccctga 3250
   tggatgaaga agacatggac gacgtggtgg atgccgacga gtacctcatc 3300
   ccacagcagg gcttcttcag cagcccctcc acgtcacgga ctcccctcct 3350
   gagctctctg agtgcaacca gcaacaattc caccgtggct tgcattgata 3400
   gaaatgggct gcaaagctgt cccatcaagg aagacagctt cttgcagcga 3450
   tacagctcag accccacagg cgccttgact gaggacagca tagacgacac 3500
   cttcctccca gtgcctgaat acataaacca gtccgttccc aaaaggcccg 3550
   ctggctctgt gcagaatcct gtctatcaca atcagcctct gaaccccgcg 3600
   cccagcagag acccacacta ccaggacccc cacagcactg cagtgggcaa 3650
   ccccgagtat ctcaacactg tccagcccac ctgtgtcaac agcacattcg 3700
   acagccctgc ccactgggcc cagaaaggca gccaccaaat tagcctggac 3750
   aaccctgact accagcagga cttctttccc aaggaagcca agccaaatgg 3800
   catctttaag ggctccacag ctgaaaatgc agaataccta agggtcgcgc 3850
   cacaaagcag tgaatttatt ggagcatgac cacggaggat agtatgagcc 3900
   ctaaaaatcc agactctttc gatacccagg accaagccac agcaggtcct 3950
   ccatcccaac agccatgccc gcattagctc ttagacccac agactggttt 4000
   tgcaacgttt acaccgacta gccaggaagt acttccacct cgggcacatt 4050
   ttgggaagtt gcattccttt gtcttcaaac tgtgaagcat ttacagaaac 4100
   gcatccagca agaatattgt ccctttgagc agaaatttat ctttcaaaga 4150
   ggtatatttg aaaaaaaaaa aaagtatatg tgaggatttt tattgattgg 4200
   ggatcttgga gtttttcatt gtcgctattg atttttactt caatgggctc 4250
   ttccaacaag gaagaagctt gctggtagca cttgctaccc tgagttcatc 4300
   caggcccaac tgtgagcaag gagcacaagc cacaagtctt ccagaggatg 4350
   cttgattcca gtggttctgc ttcaaggctt ccactgcaaa acactaaaga 4400
   tccaagaagg ccttcatggc cccagcaggc cggatcggta ctgtatcaag 4450
   tcatggcagg tacagtagga taagccactc tgtcccttcc tgggcaaaga 4500
   agaaacggag gggatggaat tcttccttag acttactttt gtaaaaatgt 4550
   ccccacggta cttactcccc actgatggac cagtggtttc cagtcatgag 4600
   cgttagactg acttgtttgt cttccattcc attgttttga aactcagtat 4650
   gctgcccctg tcttgctgtc atgaaatcag caagagagga tgacacatca 4700
   aataataact cggattccag cccacattgg attcatcagc atttggacca 4750
   atagcccaca gctgagaatg tggaatacct aaggatagca ccgcttttgt 4800
   tctcgcaaaa acgtatctcc taatttgagg ctcagatgaa atgcatcagg 4850
   tcctttgggg catagatcag aagactacaa aaatgaagct gctctgaaat 4900
   ctcctttagc catcacccca accccccaaa attagtttgt gttacttatg 4950
   gaagatagtt ttctcctttt acttcacttc aaaagctttt tactcaaaga 5000
   gtatatgttc cctccaggtc agctgccccc aaaccccctc cttacgcttt 5050
   gtcacacaaa aagtgtctct gccttgagtc atctattcaa gcacttacag 5100
   ctctggccac aacagggcat tttacaggtg cgaatgacag tagcattatg 5150
   agtagtgtgg aattcaggta gtaaatatga aactagggtt tgaaattgat 5200
   aatgctttca caacatttgc agatgtttta gaaggaaaaa agttccttcc 5250
   taaaataatt tctctacaat tggaagattg gaagattcag ctagttagga 5300
   gcccaccttt tttcctaatc tgtgtgtgcc ctgtaacctg actggttaac 5350
   agcagtcctt tgtaaacagt gttttaaact ctcctagtca atatccaccc 5400
   catccaattt atcaaggaag aaatggttca gaaaatattt tcagcctaca 5450
   gttatgttca gtcacacaca catacaaaat gttccttttg cttttaaagt 5500
   aatttttgac tcccagatca gtcagagccc ctacagcatt gttaagaaag 5550
   tatttgattt ttgtctcaat gaaaataaaa ctatattcat ttccactcta 5600
   aaaaaaaaaa aaaaaa 5616
<210> 3
   <211> 6
   <212> PRT
   <213> Homo sapiens
<400> 3
   Lys Val Leu Gly Ser Gly
   1               5
<210> 4
   <211> 18
   <212> DNA
   <213> Homo sapiens
<400> 4 $
   aaagtgctgg gctccggt 18
<210> 5
   <211> 18
   <212> DNA
   <213> Homo sapiens
<400> 5
   aaagtgctgt gctccggt 18
<210> 6
   <211> 6
   <212> PRT
   <213> Homo sapiens
<400> 6
   Lys Val Leu Ala Ser Gly
   1 5
<210> 7
   <211> 18
   <212> DNA
   <213> Homo sapiens
<400> 7
   aaagtgctgg cctccggt 18
<210> 8
   <211> 6
   <212> PRT
   <213> Homo sapiens
<400> 8
   Lys Val Leu Cys Ser Gly
   1 5
<210> 9
   <211> 19
   <212> PRT
   <213> Homo sapiens
<400> 9
   Ala Ile Lys Glu Leu Arg Glu Ala Thr Ser Pro Lys Ala Asn Lys
   1 5 10 15
   Glu Ile Leu Asp
<210> 10
   <211> 57
   <212> DNA
   <213> Homo sapiens
<400> 10
   gctatcaagg aattaagaga agcaacatct ccgaaagcca acaaggaaat 50
   cctcgat 57
<210> 11
   <211> 42
   <212> DNA
   <213> Homo sapiens
<400> 11
   gctatcaaaa catctccgaa agccaacaag gaaatcctcg at 42
<210> 12
   <211> 48
   <212> DNA
   <213> Homo sapiens
<400> 12
   gctatcaagc aaccaacatc tccgaaagcc aacaaggaaa tcctcgat 48
<210> 13
   <211> 39
   <212> DNA
   <213> Homo sapiens
<400> 13
   gctatcaagg aatcgaaagc caacaaggaa atcctcgat 39
<210> 14
   <211> 33
   <212> DNA
   <213> Homo sapiens
<400> 14
   gctatcaagg aattaagaga agcaaccctc gat 33
<210> 15
   <211> 57
   <212> DNA
   <213> Homo sapiens
<400> 15
   gctatcaagg aattaagaga agcaacatct ccgaaagtca acaaggaaat 50
   cctcgat 57
<210> 16
   <211> 57
   <212> DNA
   <213> Homo sapiens
<400> 16
   gctatcaagg aatcaagaga agcaacatct ccgaaagcca acaaggaaat 50
   cctcgat 57
<210> 17
   <211> 57
   <212> DNA
   <213> Homo sapiens
<400> 17
   gctatcaaga aattaagaga agcaacatct ccgaaagcca acaaggaaat 50
   cctcgat 57
<210> 18
   <211> 7
   <212> PRT
   <213> Homo sapiens
<400> 18
   Met Ala Ser Val Asp Asn Pro
   1 5
<210> 19
   <211> 21
   <212> DNA
   <213> Homo sapiens
<400> 19
   atggccagcg tggacaaccc c 21
<210> 20
   <211> 27
   <212> DNA
   <213> Homo sapiens
<400> 20
   atggccatag ccagcgtgga caacccc 27
<210> 21
   <211> 9
   <212> PRT
   <213> Homo sapiens
<400> 21
   Met Ala Ile Ala Ser Val Asp Asn Pro
   1 5
<210> 22
   <211> 30
   <212> DNA
   <213> Homo sapiens
<400> 22
   atggccagcg tggccagcgt ggataacccc 30
<210> 23
   <211> 10
   <212> PRT
   <213> Homo sapiens
<400> 23
   Met Ala Ser Val Ala Ser Val Asp Asn Pro
   1 5 10
<210> 24
   <211> 21
   <212> DNA
   <213> Homo sapiens
<400> 24
   atggccatcg tggacaaccc c 21
<210> 25
   <211>
   <212> PRT
   <213> Homo sapiens
<400> 25
<210> 26
   <211> 6
   <212> PRT
   <213> Homo sapiens
<400> 26
   Phe Gly Leu Ala Lys Leu
   1 5
<210> 27
   <211> 18
   <212> DNA
   <213> Homo sapiens
<400> 27
   tttgggctgg ccaaactg 18
<210> 28
   <211> 18
   <212> DNA
   <213> Homo sapiens
<400> 28
   tttgggcggg ccaaactg 18
<210> 29
   <211> 6
   <212> PRT
   <213> Homo sapiens
<400> 29
   Phe Gly Arg Ala Lys Leu
   1 5
<210> 30
   <211> 18
   <212> DNA
   <213> Homo sapiens
<400> 30
   tttgggccgg ccaaactg 18
<210> 31
   <211> 6
   <212> PRT
   <213> Homo sapiens
<400> 31
   Phe Gly Pro Ala Lys Leu
   1 5
<210> 32
   <211> 10
   <212> DNA
   <213> Homo sapiens
<400> 32
   ggaattaaga 10
<210> 33
   <211> 15
   <212> DNA
   <213> Homo sapiens
<400> 33
   ggaattaaga gaagc 15
<210> 34
   <211> 18
   <212> DNA
   <213> Homo sapiens
<400> 34
   taagagaagc aacatctc 18
<210> 35
   <211> 18
   <212> RNA
   <213> Homo sapiens
<400> 35
   ttaagagaag caacatct 18
<210> 36
   <211> 12
   <212> DNA
   <213> Homo sapiens
<400> 36
   taagagaagc aa 12
<210> 37
   <211> 24
   <212> DNA
   <213> Homo sapiens
<400> 37
   atctccgaaa gccaacaagg aaat 24
<210> 38
   <211> 19
   <212> PRT
   <213> Homo sapiens
<400> 38
   Lys Lys Lys Gly Pro Trp Leu Glu Glu Glu Glu Glu Ala Tyr Gly
   1 5 10 15
   Trp Leu Asp Phe
<210> 39
   <211> 25
   <212> DNA
   <213> Homo sapiens
<400> 39
   caaatgagct ggcaagtgcc gtgtc 25
<210> 40
   <211> 25
   <212> DNA
   <213> Homo sapiens
<400> 40
   gagtttccca aacactcagt gaaac 25
<210> 41
   <211> 25
   <212> DNA
   <213> Homo sapiens
<400> 41
   gcaatatcag ccttaggtgc ggctc 25
<210> 42
   <211> 26
   <212> DNA
   <213> Homo sapiens
<400> 42
   catagaaagt gaacatttag gatgtg 26
<210> 43
   <211> 24
   <212> DNA
   <213> Homo sapiens
<400> 43
   ccatgagtac gtattttgaa actc 24
<210> 44
   <211> 24
   <212> DNA
   <213> Homo sapiens
<400> 44
   catatcccca tggcaaactc ttgc 24
<210> 45
   <211> 24
   <212> DNA
   <213> Homo sapiens
<400> 45
   ctaacgttcg ccagccataa gtcc 24
<210> 46
   <211> 26
   <212> DNA
   <213> Homo sapiens
<400> 46
   gctgcgagct cacccagaat gtctgg 26
<210> 47
   <211> 44
   <212> DNA
   <213> Homo sapiens
<400> 47
   tgtaaaacga cggccagtca agtgccgtgt cctggcaccc aagc 44
<210> 48
   <211> 42
   <212> DNA
   <213> Homo sapiens
<400> 48
   caggaaacag ctatgacccc aaacactcag tgaaacaaag ag 42
<210> 49
   <211> 39
   <212> DNA
   <213> Homo sapiens
<400> 49
   tgtaaaacga cggccagtcc ttaggtgcgg ctccacagc 39
<210> 50
   <211> 40
   <212> DNA
   <213> Homo sapiens
<400> 50
   caggaaacag ctatgaccca tttaggatgt ggagatgagc 40
<210> 51
   <211> 41
   <212> DNA
   <213> Homo sapiens
<400> 51
   tgtaaaacga cggccagtga aactcaagat cgcattcatg c 41
<210> 52
   <211> 41
   <212> DNA
   <213> Homo sapiens
<400> 52
   caggaaacag ctatgaccgc aaactcttgc tatcccagga g 41
<210> 53
   <211> 40
   <212> DNA
   <213> Homo sapiens
<400> 53
   -tgtaaaacga-cggccagtca gccataagtc ctcgacgtgg 40
<210> 54
   <211> 42
   <212> DNA
   <213> Homo sapiens
<400> 54
   caggaaacag ctatgaccca tcctcccctg catgtgttaa ac 42
<210> 55
   <211> 23
   <212> DNA
   <213> Homo sapiens
<400> 55
   tactggtgga gtatttgata gtg 23
<210> 56
   <211> 21
   <212> DNA
   <213> Homo sapiens
<400> 56
   ctgtatcaaa gaatggtcct g 21
<210> 57
   <211> 38
   <212> DNA
   <213> Homo sapiens
<400> 57
   tgtaaaacga cggccagtta gtgtattaac cttatgtg 38
<210> 58
   <211> 42
   <212> DNA
   <213> Homo sapiens
<400> 58
   caggaaacag ctatgaccac ctctattgtt ggatcatatt cg 42
<210> 59
   <211> 18
   <212> DNA
   <213> Homo sapiens
<400> 59
   tgtaaaacga cggccagt 18
<210> 60
   <211> 18
   <212> DNA
   <213> Homo sapiens
<400> 60
   caggaaacag ctatgacc 18